# EUROPÄISCHE PATENTANMELDUNG

(11) **EP 1 681 046 A2**
(43) Veröffentlichungstag der Anmeldung: **19.07.2006**
(21) Anmeldenummer: 05023824.5
(22) Anmeldetag: 02.11.2005
(51) Int. Cl.: A61K 8/18

(54) **Kosmetische, pharmazeutische und dermatologische Zubereitungen enthaltend Copolymerwachse**

(30) Priorität: 13.11.2004 DE 102004054849; 24.02.2005 DE 102005008442
(71) Anmelder: Clariant Produkte (Deutschland) GmbH, 65929 Frankfurt am Main (DE)
(72) Erfinder: Heinrichs, Franz-Leo, Dr., 86456 Gablingen (DE); Lukasch, Anton, Dr., 86405 Meitingen (DE); Hohner, Gerd, Dr., 86368 Gersthofen (DE); Michaelis, Heike, 64295 Darmstadt (DE); Lachmann, Angela, Dr., 65779 Kelkheim-Fischbach (DE)

(57) **Zusammenfassung**

Es werden kosmetische, pharmazeutische und dermatologische Zubereitungen beschrieben, die Copolymerwachse enthalten. Die Copolymerwachse enthalten Struktureinheiten, die formell abgeleitet sind von α-Olefinen mit 26 bis 60 Kohlenstoffatomen, Derivaten von (Meth)acrylsäure wie Estern, Amiden oder Salzen und gegebenenfalls weiteren Monomeren.

## Beschreibung

Die Erfindung betrifft kosmetische, pharmazeutische und dermatologische Zubereitungen, enthaltend Copolymerwachse aus langkettigen α-Olefinen, Derivaten von (Meth)acrylsäure und gegebenenfalls weiteren Monomeren.

Wachse und wachsähnliche Substanzen bestimmen hauptsächlich die Konsistenz von vielen Kosmetik-Produkten. Wachse werden verwendet, um Härte und Festigkeit von kosmetischen Produkten zu beeinflussen. Je mehr besonders harte und erst bei hohen Temperaturen schmelzende Wachse eingesetzt werden, umso fester wird das Produkt. In der Kosmetik werden häufig natürliche Wachse tierischen und pflanzlichen Ursprungs, wie Bienenwachs, Beerenwachs, Rosenwachs, Japanwachs, Chinawachs, Schellackwachs, Quittenwachs, Shea-Butter, Candelillawachs, Carnaubawachs, Lanolin (Wollfett), Jojobaöl und Jojobawachs verwendet. Wegen einer Belastung natürlicher Wachse durch Pestizide sucht man nach alternativen, synthetisch hergestellten Wachsen, die frei von Pflanzenschutzmitteln und allergenen Stoffen sind.

In DE 41 39 601 werden Copolymere aus Olefinen mit Acrylsäure- und Acrylsäuremethylestern und deren Verwendung als Gleitmittel in der Kunststoffverarbeitung beschrieben.

In DE 102 25 652 wird ein Verfahren zur Herstellung seitenkettenmodifizierter Copolymerwachse aus langkettigen Olefinen, Acrylsäureestern, Acrylsäure und/oder Acrylsäureamiden und deren Verwendung als Mattierungsmittel, Slipmittel sowie Antikratzmittel in der Kunststoff- und Automobilindustrie offenbart.

In WO 2004/041220 und WO 2004/041150 werden kosmetische Zubereitungen beschrieben, insbesondere Lippenstifte und Make-ups, enthaltend neben einer flüssigen Fettphase ein semikristallines Polymer aus einem C₁₄-C₂₄-α-Olefin und einem weiteren Monomer ausgewählt aus Carbonsäureestern, vorzugsweise C₁₄-C₂₄-Alkyl- oder C₁₁-C₁₅-Perfluoroalkyl-(meth)acrylaten, und N-Alkyl(meth)-acrylamiden. Die beschriebenen Polymere sind zumindest teilweise öllöslich und sind zur Herstellung temperaturbeständiger Sticks und Pasten ungeeignet.

Es bestand die Aufgabe, Substanzen für kosmetische, pharmazeutische und dermatologische Zubereitungen zur Verfügung zu stellen, die ähnlich gute konsistenzgebende Eigenschaften wie natürlich vorkommende Wachse aufweisen, mit wässrigen Systemen und mit Ölsystemen gut verträglich sind, ein klares visuelles Erscheinungsbild haben, leicht verarbeitbar und kompatibel mit Wirksubstanzen (z.B. Sonnenschutzfiltern) sind, Temperatur- und Lagerbeständigkeit aufweisen, aber auch hautverträglich und toxikologisch unbedenklich sind.

Es wurde überraschend gefunden, dass diese Aufgabe gelöst wird durch Copolymerwachse enthaltend Struktureinheiten, die sich formell ableiten von α-Olefinen mit 26 bis 60 Kohlenstoffatomen sowie von (Meth)acrylsäureestern, (Meth)acrylsäureamiden und/oder (Meth)acrylsäuresalzen und gegebenenfalls von weiteren Monomeren und dass diese Copolymerwachse ein herausragendes Ölbindevermögen zeigen, flüssigen Ölphasen eine pastenförmige bis feste Konsistenz verleihen, sowie als Gleitmittel, Dispergierhilfsmittel und Haftmittel geeignet sind. Sie können als polare synthetische Wachse als Ersatz für natürliche Wachse in kosmetischen, pharmazeutischen und dermatologischen Zubereitungen verwendet werden. Darüber hinaus zeigen die Copolymerwachse zusätzliche wertvolle anwendungstechnische Eigenschaften. Sie haben ein weißes bis beiges Aussehen, sind fest bis brüchig hart, gut verarbeitbar und gut geeignet zur FormStabilisierung fester Mittel, beispielsweise von Sticks. Sie sind geeignet zur Viskositätseinstellung cremiger Emulsionen oder Dispersionen, sowie hydroxysäurehaltiger und elektrolythaltiger Mittel, verbessern die Feinteiligkeit bzw. Stabilität von Emulsionen und können zu fließfähigen Zubereitungen verarbeitet werden. Sie verbessern signifikant das Aufnahmevermögen von Pigmenten in der Lipidphase und die Pigmentdispergierung, sowie die Wirkung von Effektpigmenten. Sehr vorteilhaft für die kosmetische Anwendung ist das Frischegefühl auf der Haut, ein gutes Spreitungsvermögen, die Wasserfestigkeit und Haftfestigkeit der Mittel. Die Migration fester Inhaltsstoffe (z.B. Pigmente) wird unterdrückt, ebenso die Tendenz einzelner Inhaltsstoffe in die Haut zu penetrieren. Damit wird eine Reduzierung der Reizwirkung von Inhaltsstoffen erreicht. Vorteilhaft ist auch eine verzögerte Freisetzung von Wirksubstanzen, sowie eine verbesserte Kompatbilität einzelner in kosmetischen und pharmazeutischen Mitteln üblicher Komponenten.

Gegenstand der Erfindung sind kosmetische, pharmazeutische und dermatologische Zubereitungen, die ein oder mehrere Copolymerwachse, enthaltend
a) eine oder mehrere Struktureinheiten -CH₂-CHR-, wobei R für eine lineare oder verzweigte Alkylgruppe mit 24 bis 58 Kohlenstoffatomen steht,
b) gegebenenfalls eine oder mehrere Struktureinheiten 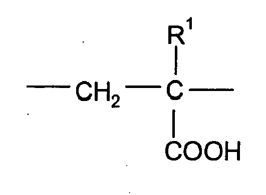 wobei R¹ für Wasserstoff oder Methyl steht,
c) eine oder mehrere Struktureinheiten 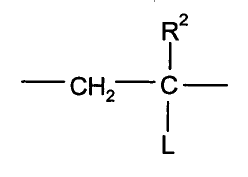 wobei
   - R²: für Wasserstoff oder Methyl steht,
   - L: -COOR³, -CONR⁷R⁸ oder -COO⁻X⁺ bedeutet,
   - R³: für eine geradkettigte oder verzweigte Alkylgruppe mit 1 bis 36 Kohlenstoffatomen oder eine geradkettigte oder verzweigte Alkenylgruppe mit 2 bis 36 Kohlenstoffatomen steht, die gegebenenfalls auch alkoxyliert sein kann und vorzugsweise Ethylenoxy- (EO), Propylenoxy- (PO), Butylenoxy- (BO) oder EO/PO-Gruppen enthalten kann, oder
   eine Gruppe (AO)ₓ-H ist, wobei (AO) für eine Ethoxy-, Propoxy- oder Butoxygruppe steht und x eine Zahl von 1 bis 50 ist, oder
   für eine Glycidylgruppe, eine C₂-C₁₀-Hydroxyalkylgruppe oder für eine Glyceringruppe steht, oder
   für eine cyclische aromatische oder nichtaromatische Gruppe, vorzugsweise eine Cycloalkylgruppe, mit 5 bis 8, vorzugsweise 6, Ringatomen steht, oder
   für eine cyclische aromatische oder nichtaromatische Gruppe mit 5 bis 8, vorzugsweise 6, Ringatomen steht, wobei der Ring aus Kohlenstoffatomen und Heteroatomen, vorzugsweise O und/oder N, gebildet ist, und sowohl die Kohlenstoffatome, als auch die Stickstoffatome mit linearen oder verzweigten Alkyl- oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁴, worin R⁴ eine Alkylgruppe mit 1 bis 22 Kohlenstoffatomen ist, substituiert sein können, oder
   für -(CR⁵R⁶)_{y}-Cycloalkyl oder für -(CR⁵R⁶)_{y}-Aryl steht, wobei R⁵ und R⁶ jeweils unabhängig voneinander für H oder eine lineare oder verzweigte Alkylgruppe mit 1 bis 4 Kohlenstoffatomen stehen und y eine Zahl von 1 bis 10 ist, oder
   für eine Perfluoralkylgruppe mit 8 bis 18 Kohlenstoffatomen steht,
   - R⁷ und R⁸: jeweils unabhängig voneinander
   für Wasserstoff stehen, oder
   für eine geradkettigte oder verzweigte Alkylgruppe mit 1 bis 36 Kohlenstoffatomen oder eine geradkettigte oder verzweigte Alkenylgruppe mit 2 bis 36 Kohlenstoffatomen stehen, die gegebenenfalls auch alkoxyliert sein können und vorzugsweise Ethylenoxy- (EO), Propylenoxy- (PO), Butylenoxy- (BO) oder EO/PO- Gruppen enthalten können, oder
   für eine (C₂-C₁₀)-Hydroxyalkylgruppe stehen, oder
   für -CH₂-CH₂-N(CH₃)₂ oder für einen Polyaminrest stehen, oder
   für eine cyclische aromatische oder nichtaromatische Gruppe, vorzugsweise eine Cycloalkylgruppe, mit 5 bis 8, vorzugsweise 6, Ringatomen stehen, oder
   für eine cyclische aromatische oder nichtaromatische Gruppe mit 5 bis 8, vorzugsweise 6, Ringatomen stehen, wobei die Ringe aus Kohlenstoffatomen und Heteroatomen, vorzugsweise O und/oder N, gebildet sind, und sowohl die Kohlenstoffatome, als auch die Stickstoffatome mit linearen oder verzweigten Alkyl- oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁹, worin R⁹ eine Alkylgruppe mit 1 bis 22 Kohlenstoffatomen ist, substituiert sein können, oder
   R⁷ und R⁸ zusammen mit dem Stickstoffatom, an das sie gebunden sind, einen 5-, 6- oder 7-gliedrigen aromatischen oder nichtaromatischen Ring bilden, und die Ringe neben dem Stickstoffatom vorzugsweise lediglich CH₂-Gruppen enthalten,
   - X⁺: für Li⁺, Na⁺, K⁺, Mg⁺⁺/2, Ca⁺⁺/2, Al⁺⁺⁺/3, NH₄⁺, ein Monoalkylammonium-, Dialkylammonium-, Trialkylammonium- und/oder Tetraalkylammoniumion steht, wobei es sich bei den Alkylsubstituenten der Ammoniumionen unabhängig voneinander um (C₁-C₂₂)-Alkylreste oder (C₂-C₁₀)-Hydroxyalkylreste handeln kann, und
d) gegebenenfalls eine oder mehrere Struktureinheiten, die sich von Styrol, 3-Methylstyrol, 4-Methylstyrol oder α-Methylstyrol ableiten,
enthalten.

Die Struktureinheiten der Komponente a) gehen durch Polymerisation von C₂₆-C₆₀-α-Olefinen hervor.

Die Struktureinheiten der Komponente b) gehen durch Polymerisation von ungesättigten Carbonsäuren CH₂=CR¹-COOH, worin R¹ Wasserstoff oder Methyl bedeutet, hervor.
Die Struktureinheiten der Komponente c) gehen z.B. durch Polymerisation von ungesättigten Verbindungen der Formel CH₂=CR²-L, worin R² und L die oben unter Komponente c) angegebenen Bedeutungen besitzen, hervor.

Die Struktureinheiten der Komponente c) können z.B. auch erhalten werden, wenn zunächst die ungesättigten Verbindungen CH₂=CR²-COOMe, worin R² Wasserstoff oder Methyl und Me Methyl bedeutet, copolymerisiert werden und die dabei entstehenden Struktureinheiten 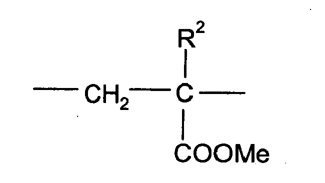 nach der Polymerisation durch entsprechende Umsetzung, z.B. mit nucleophilen Reagenzien, in die Struktureinheiten 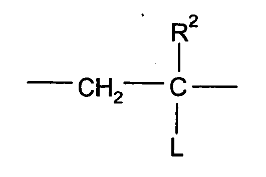 umgewandelt werden. Sofern die Copolymere auch Struktureinheiten der Komponente b) enthalten, kann nicht ausgeschlossen werden, dass auch diese Struktureinheiten bei der genannten Umsetzung der Methylester zumindest teilweise mit umgewandelt werden. Zubereitungen, die derartige Copolymere enthalten, sind ebenfalls erfindungsgemäß.

Die Struktureinheiten der Komponente d) haben folgendes Aussehen:

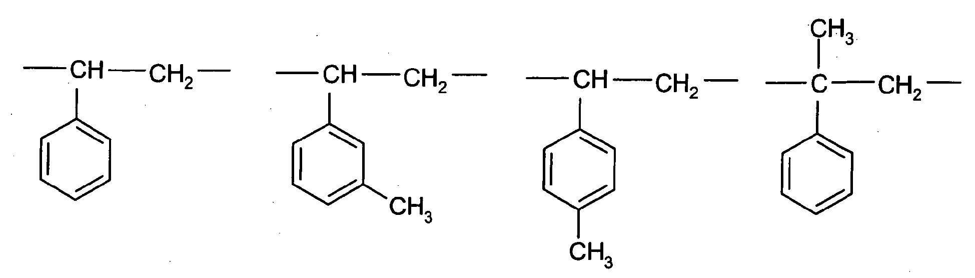

Das Eigenschaftsprofil der erfindungsgemäß eingesetzten Copolymerwachse kann durch die Komponenten a) bis d), sowie durch deren Mengenverhältnisse beeinflußt werden. Es können Copolymerwachse mit Säurezahlen von 5 bis 60, vorzugsweise von 5 bis 30 und besonders bevorzugt von 5 bis 15 und Esterzahlen von bis zu ca. 290 hergestellt werden. Die Schmelzen sind klar und transparent. Die Tropfpunkte fallen mit steigendem Gehalt an Struktureinheiten der Komponenten b) bis d) geringfügig ab und liegen bei Copolymeren mit hohem Acrylsäure- und/oder Methacrylsäureanteil im Bereich von 65 bis 75°C. Die Viskosität nimmt mit steigendem Gehalt an Struktureinheiten der Komponenten b) bis d), insbesondere mit steigendem Acrylsäureanteil, stark zu, ebenso nehmen die Fließhärten zu. Schmelzwärme und Kristallinität nehmen mit wachsendem Gehalt an Struktureinheiten der Komponenten b) bis d) ab.

In einer bevorzugten Ausführungsform enthalten die erfindungsgemäßen Zubereitungen Copolymerwachse, die Struktureinheiten der Komponente a) enthalten, worin R für eine lineare oder verzweigte Alkylgruppe mit 24 und/oder 26 Kohlenstoffatomen steht. Diese Struktureinheiten der Komponente a) sind abgeleitet von C_{26/28}-α-Olefinen. Es werden Copolymerwachse mit Schmelzpunkten von 30 bis 80°C erhalten, die ein gutes Spreitungsvermögen und eine gute Hautsensorik zeigen. Beim Auftragen dieser Copolymerwachse auf die Haut oder auf die Haare entstehen wasserabweisende Filme, die die Migration und das Aus- und Abwaschen von Inhaltsstoffen der kosmetischen, pharmazeutischen und dermatologischen Mittel, insbesondere von Wirksubstanzen und/oder Pigmenten, verhindern.

In einer weiteren bevorzugten Ausführungsform enthalten die erfindungsgemäßen Zubereitungen Copolymerwachse, die Struktureinheiten der Komponente a) enthalten, worin R für eine lineare oder verzweigte Alkylgruppe mit 28 bis 58 Kohlenstoffatomen steht.

Diese Struktureinheiten der Komponente a) sind abgeleitet von C₃₀₊-a-Olefinen. Es werden Copolymerwachse mit Schmelzpunkten von 35 bis 75°C erhalten, die sich durch eine gute Hautsensorik auszeichnen. Zudem werden härtere Wachse erhalten, die ein sehr gutes Ölbindevermögen zeigen und mit der flüssigen Lipidphase zu festen, temperaturbeständigen Mitteln, beispielsweise Lippenstiften, verarbeitet werden können.

In besonders bevorzugten Ausführungsformen enthalten die erfindungsgemäßen Zubereitungen Copolymerwachse, die Struktureinheiten der Komponente a) enthalten, worin R für eine lineare oder verzweigte Alkylgruppe
- mit 28 und/oder 30 und/oder 32 Kohlenstoffatomen steht, oder
- mit 30 und/oder 32 und/oder 34 Kohlenstoffatomen steht, oder
- mit 32 und/oder 34 und/oder 36 Kohlenstoffatomen steht, oder
- mit 34 und/oder 36 und/oder 38 Kohlenstoffatomen steht, oder
- mit 36 und/oder 38 und/oder 40 Kohlenstoffatomen steht.

In einer weiteren bevorzugten Ausführungsform enthalten die erfindungsgemäßen Zubereitungen Copolymerwachse, die Struktureinheiten der Komponente c) enthalten, worin R² Wasserstoff oder Methyl, vorzugsweise Wasserstoff, bedeutet, L -COOR³ ist und R³ für eine cyclische aromatische oder nichtaromatische Gruppe mit 5 bis 8, vorzugsweise 6, Ringatomen steht, wobei der Ring aus Kohlenstoffatomen und Heteroatomen, vorzugsweise O und/oder N, gebildet ist, und sowohl die Kohlenstoffatome, als auch die Stickstoffatome mit linearen oder verzweigten Alkyl- oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁴, worin R⁴ eine Alkylgruppe mit 1 bis 22 Kohlenstoffatomen ist, substituiert sein können.

Unter diesen Copolymerwachsen sind wiederum diejenigen bevorzugt, in denen R³ ein Rest ist, der von Tetramethylpiperidinol, 2,2,6,6-Tetramethylpiperidinol, N-Methyl-2,2,6,6-tetramethylpiperidinol, N-Acetyl-2,2,6,6-tetramethylpiperidinol und/oder 2,2,6,6-Tetramethylpiperidinol-N-Oxid abgeleitet ist.

Bei 2,2,6,6-Tetramethylpiperidinol handelt es sich beispielsweise um die folgende Substanz

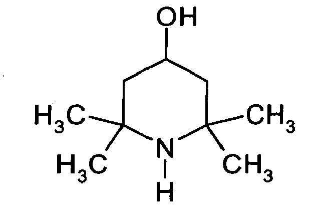

In einer weiteren bevorzugten Ausführungsform enthalten die erfindungsgemäßen Zubereitungen Copolymerwachse, die Struktureinheiten der Komponente c) enthalten, worin R² Wasserstoff oder Methyl, vorzugsweise Wasserstoff, bedeutet, L COOR³ ist und R³ für eine geradkettige oder verzweigte Alkylgruppe mit 1 bis 36 Kohlenstoffatomen oder eine geradkettige oder verzweigte Alkenylgruppe mit 2 bis 36 Kohlenstoffatomen steht, die gegebenenfalls auch alkoxyliert sein kann, vorzugsweise mit 1 bis 30 Alkoxyeinheiten, und insbesondere ethoxyliert, propoxyliert, butoxyliert oder gleichzeitig ethoxyliert/propoxyliert sein kann.

In einer besonders bevorzugten Ausführungsform enthalten die erfindungsgemäßen Zubereitungen Copolymerwachse, die Struktureinheiten der Komponente c) enthalten, worin R² Wasserstoff oder Methyl, vorzugsweise Wasserstoff, bedeutet, L COOR³ ist und R³ für eine geradkettige oder verzweigte Alkylgruppe mit 1 bis 13, vorzugsweise mit 1 bis 10 und besonders bevorzugt mit 1 bis 8 Kohlenstoffatomen.

Unter diesen Copolymerwachsen sind wiederum diejenigen bevorzugt, die erhalten werden durch Copolymerisation von C₂₆₋₆₀-α-Olefinen, bevorzugt C_{26/28}-α-Olefinen oder C₃₀₊-α-Olefinen, gegebenenfalls (Meth)acrylsäure, und (Meth)acrylsäuremethylester, wobei die Methylesterfunktionen nach der Copolymerisation durch Umsetzung mit kurzkettigen Alkoholen, vorzugsweise Ethanol, Propanol, Isopropanol, n-Butanol, i-Butanol, tert.-Butanol, Hexanol und/oder Laurylalkohol umgeestert wurden.

Die Copolymerwachse mit kurzen Alkylketten in der Estergruppe der Komponente c) zeichnen sich durch geringere Erweichungspunkte, durch ein gutes Dispergiervermögen und durch einen amorphen glasartigen Charakter aus. Die Kristallisationsneigung ist unterdrückt.

In einer weiteren besonders bevorzugten Ausführungsform enthalten die erfindungsgemäßen Zubereitungen Copolymerwachse, die Struktureinheiten der Komponente c) enthalten, worin R² Wasserstoff oder Methyl, vorzugsweise Wasserstoff, bedeutet, L COOR³ ist und R³ für eine geradkettige oder verzweigte Alkylgruppe mit 14 bis 36, vorzugsweise mit 14 bis 30 und besonders bevorzugt mit 14 bis 22 Kohlenstoffatomen steht. Diese Alkylgruppen können gegebenenfalls auch alkoxyliert sein.

Unter diesen Copolymerwachsen sind wiederum diejenigen bevorzugt, die erhalten werden durch Copolymerisation von C₂₆₋₆₀-α-Olefinen, bevorzugt C_{26/28}-α-Olefinen oder C₃₀₊-α-Olefinen, gegebenenfalls (Meth)acrylsäure, und (Meth)acrylsäuremethylester, wobei die Methylesterfunktionen nach der Copolymerisation durch Umsetzung mit langkettigen Alkoholen, die gegebenenfalls auch alkoxyliert sein können, vorzugsweise Talgfettalkohol, Myristylalkohol, Palmitylalkohol, Stearylalkohol, Kokosfettalkohol, Guerbetalkohol und/oder Behenylalkohol umgeestert wurden.

Durch Einbau langer Kohlenstoffketten in die Estergruppe der Komponente c) lassen sich Copolymerwachse mit ausgezeichnetem Ölbindevermögen herstellen. Copolymerwachse mit langen Alkylketten in der Estergruppe haben zudem eine hohe Kristallinität und eine bessere Härte.

In einer weiteren bevorzugten Ausführungsform enthalten die erfindungsgemäßen Zubereitungen Copolymerwachse, die erhalten werden durch Copolymerisation von C₂₆₋₆₀-α-Olefinen, bevorzugt C_{26/28}-α-Olefinen oder C₃₀₊-α-Olefinen, gegebenenfalls (Meth)acrylsäure, und (Meth)acrylsäuremethylester, wobei die Methylesterfunktionen nach der Copolymerisation durch Umsetzung mit Glycerin, Alkylenglykolen, vorzugsweise Propylen-, Butylen- oder Hexylenglykol, und/oder entsprechenden Polyalkylenglykolen modifiziert wurden.

In einer weiteren bevorzugten Ausführungsform enthalten die erfindungsgemäßen Zubereitungen Copolymerwachse, die Struktureinheiten der Komponente c) enthalten, worin R² Wasserstoff oder Methyl, vorzugsweise Wasserstoff, bedeutet, L COOR³ ist und R³ für eine Perfluoralkylgruppe mit 8 bis 18 Kohlenstoffatomen steht.

Diese Copolymerwachse zeichnen sich durch besondere wasserabweisende Eigenschaften aus.

In einer weiteren bevorzugten Ausführungsform enthalten die erfindungsgemäßen Zubereitungen Copolymerwachse, die Struktureinheiten der Komponente c) enthalten, worin R² Wasserstoff oder Methyl, vorzugsweise Wasserstoff, bedeutet, L -CONR⁷R⁸ ist und R⁷ und R⁸ jeweils unabhängig voneinander
für Wasserstoff stehen, oder
für eine geradkettigte oder verzweigte Alkylgruppe mit 1 bis 36 Kohlenstoffatomen oder eine geradkettigte oder verzweigte Alkenylgruppe mit 2 bis 36 Kohlenstoffatomen stehen, die gegebenenfalls auch alkoxyliert sein können und vorzugsweise Ethylenoxy- (EO), Propylenoxy- (PO), Butylenoxy-(BO) oder EO/PO-Gruppen enthalten können, oder
für eine (C₂-C₁₀)-Hydroxyalkylgruppe stehen, oder
für -CH₂-CH₂-N(CH₃)₂ oder für einen Polyaminrest stehen, oder
für eine cyclische aromatische oder nichtaromatische Gruppe, vorzugsweise eine Cycloalkylgruppe, mit 5 bis 8, vorzugsweise 6, Ringatomen stehen, oder für eine cyclische aromatische oder nichtaromatische Gruppe mit 5 bis 8, vorzugsweise 6, Ringatomen stehen, wobei die Ringe aus Kohlenstoffatomen und Heteroatomen, vorzugsweise O und/oder N, gebildet sind, und sowohl die Kohlenstoffatome, als auch die Stickstoffatome mit linearen oder verzweigten Alkyl- oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁹, worin R⁹ eine Alkylgruppe mit 1 bis 22 Kohlenstoffatomen ist, substituiert sein können, oder
R⁷ und R⁸ zusammen mit dem Stickstoffatom, an das sie gebunden sind, einen 5-, 6- oder 7-gliedrigen aromatischen oder nichtaromatischen Ring bilden, und die Ringe neben dem Stickstoffatom vorzugsweise lediglich CH₂₋Gruppen enthalten.

In einer weiteren bevorzugten Ausführungsform enthalten die erfindungsgemäßen Zubereitungen Copolymerwachse, die erhalten werden durch Copolymerisation von C₂₆₋₆₀-α-Olefinen, bevorzugt C_{26/28}-α-Olefinen oder C₃₀₊-α-Olefinen, gegebenenfalls (Meth)acrylsäure, und (Meth)acrylsäuremethylester, wobei die Methylesterfunktionen nach der Copolymerisation durch Umsetzung mit entsprechenden Aminen wie z.B. kurzkettigen Aminen oder langkettigen Aminen und/oder Aminalkoholen modifiziert wurden. Bevorzugte Amine und Aminalkohole sind Butylamin, Dimethylaminopropylamin, Triacetondiamin, Octylamin, Decylamin, Dodecylamin, Talgfettamin, Kokosfettamin, Didecylamin, Cyclohexylamin und/oder Diethylaminoethanol.

Diese Copolymerwachse zeichnen sich durch eine besonders gute Substantivität auf der Haut und auf den Haaren aus.

Bei den erfindungsgemäß eingesetzten Copolymerwachsen handelt es sich um Kammpolymere auf Basis von langkettigen α-Olefinen und ethylenisch ungesättigten Säurederivaten und gegebenenfalls weiteren Monomeren. Die polaren und unpolaren Anteile sind zufällig angeordnet. Bei hohem Anteil kurzer Seitenketten überwiegt der amorphe, glasartige Charakter, bei hohem Anteil langer Ketten überwiegt der kristalline Wachscharakter. C_{26/28}-α-Olefin ist reaktiver als C₃₀₊-α-Olefin und setzt sich zu höhermolekularen Verbindungen um. Das resultierende Produkt ist glasartiger. Ein hoher Anteil an Estermonomeren erhöht die Viskosität. Durch Modifizierung der Monomeranteile der Copolymerwachse, aber auch durch Umesterung, Amidierung oder Verseifung können die physikalischen Eigenschaften der Copolymerwachse, beispielsweise Spreitvermögen, Emulgierung, Ölverträglichkeit, Schmelzpunkt, Viskosität und/oder Kristallinität verändert werden.

In einer weiteren bevorzugten Ausführungsform enthalten die erfindungsgemäßen Zubereitungen Copolymerwachse, die eine oder mehrere Struktureinheiten der Komponente b) enthalten, worin R¹ für Wasserstoff oder Methyl und vorzugsweise für Wasserstoff steht.

Die in den erfindungsgemäßen Zubereitungen enthaltenen Copolymerwachse enthalten die Komponenten a), b) und c) in den molaren Verhältnissen
Komponente a) : Komponente b) : Komponente c) von
vorzugsweise 1:0-2:1-4,
besonders bevorzugt 1 : 0 - 0,5 : 1,5 - 3 und
insbesondere bevorzugt 1 : 0,1 - 0,25 : 2,0 - 2,5.

Die in den erfindungsgemäßen Zubereitungen enthaltenen Copolymerwachse, in denen sich die Struktureinheiten der Komponente a) von C_{26/28}-α-Olefin ableiten, enthalten die Komponenten a), b) und c) in den molaren Verhältnissen
Komponente a) : Komponente b): Komponente c) von
vorzugsweise 1 : 0 - 1 : 1 - 3,
besonders bevorzugt 1 : 0 - 0,5 : 1,5 - 3 und
insbesondere bevorzugt 1 : 0,1 - 0,25 : 2,0 - 2,5.

Die in den erfindungsgemäßen Zubereitungen enthaltenen Copolymerwachse, in denen sich die Struktureinheiten der Komponente a) von C₃₀₊-α-Olefin ableiten, enthalten die Komponenten a), b) und c) in den molaren Verhältnissen
Komponente a) : Komponente b) : Komponente c) von
vorzugsweise 1 : 0 - 2 : 1 - 4,
besonders bevorzugt 1 : 0 - 0,5 : 1,5 - 3 und
insbesondere bevorzugt 1 : 0,1 - 0,25 : 2,25 - 2,4.

Die erfindungsgemäß eingesetzten Copolymerwachse haben Molekulargewichte vorzugsweise im Bereich von 1 000 bis 500 000, besonders bevorzugt im Bereich von 1 500 bis 150 000 und insbesondere bevorzugt im Bereich von 1 500 bis 100 000.

In einer weiteren bevorzugten Ausführungsform enthalten die erfindungsgemäßen Zubereitungen Copolymerwachse, die aus Struktureinheiten der Komponenten a) und c) bestehen.

In einer weiteren bevorzugten Ausführungsform enthalten die erfindungsgemäßen Zubereitungen Copolymerwachse, die Struktureinheiten der Komponenten a), b) und c) enthalten. In einer besonders bevorzugten Ausführungsform enthalten die Copolymerwachse keine weiteren Struktureinheiten, d.h. sie bestehen aus den Struktureinheiten der Komponenten a), b) und c). In einer weiteren bevorzugten Ausführungsform enthalten die erfindungsgemäßen Zubereitungen Copolymerwachse, die Struktureinheiten der Komponenten a), c) und d) enthalten. In einer besonders bevorzugten Ausführungsform enthalten die Copolymerwachse keine weiteren Struktureinheiten, d.h. sie bestehen aus den Struktureinheiten der Komponenten a), c) und d).

In einer weiteren bevorzugten Ausführungsform enthalten die erfindungsgemäßen Zubereitungen Copolymerwachse, die Struktureinheiten der Komponenten a), b), c) und d) enthalten. In einer besonders bevorzugten Ausführungsform enthalten die Copolymerwachse keine weiteren Struktureinheiten, d.h. sie bestehen aus den Struktureinheiten der Komponenten a), b), c) und d).

In einer weiteren bevorzugten Ausführungsform enthalten die erfindungsgemäßen Zubereitungen Copolymerwachse, deren Struktureinheiten der Komponenten a) und c) sowie gegebenenfalls b) und d) aus den als bevorzugt angegebenen Struktureinheiten bestehen, d.h. in diesen Fällen enthalten die Copolymerwachse keine weiteren Struktureinheiten der jeweiligen Komponenten.

Die erfindungsgemäß eingesetzten Copolymerwachse aus C₂₆₋₆₀-α-Olefinen mit (Meth)acrylsäure und (Meth)acrylsäuremethylester können nach den in EP 545 306 offenbarten Verfahren hergestellt werden. Die seitenkettenmodifizierten Copolymere können nach den in DE 102 25 652 beschriebenen Methoden erhalten werden.

Bei den erfindungsgemäßen Mitteln kann es sich um die unterschiedlichsten kosmetischen, pharmazeutischen und dermatologischen Zubereitungen handeln. Insbesondere kann es sich um Abdeckstifte, Akne-Stifte, Lippenstifte, Make-ups, Grundierungen, Gesichtspuder, Rouge, Mascara, Lidschatten, Eye-liner, Peeling-Cremes, Haarwachs, Haar-Stylingmittel, Styling-Fluid, Haarschaum, Haargel, Haarspray, Mousse, Haarölen und Spitzenfluids, Haarkuren, Nachtcremes, Pflegecremes, Nährcremes, Bodylotions, Salben oder Lippenpflegemittel, Sonnenschutzmittel, Deodorantien, Antiperspirantien und colorierte Gele in Form von Stiften, Mehrphasenstiften, Sticks, Pasten, Pudern, Cremes, Cremeschäume, Lotionen, selbstschäumenden, schaumförmigen, nachschäumenden oder schäumbaren Emulsionen, Gelen, Roll-On-Präparaten und Schäumen handeln. Die oben beschriebenen Copolymerwachse sind geruchsneutral, weiß bis beige und haben ausgezeichnete Verarbeitungseigenschaften. Sie sind leicht emulgierbar und daher zur Herstellung stabiler Wachsemulsionen verschiedenster Art sehr gut geeignet. Sie sind ausgezeichnet geeignet als Hartwachs für kosmetische Stifte und als Konsistenzgeber. Sie besitzen gute Absorptionseigenschaften, die u.a. für die Absorption von Ölen und die Dispersion von Pigmenten, Geruchsstoffen oder festen Wirkstoffen genutzt werden können.

Aufgrund ihrer Härte verleihen die in den erfindungsgemäßen Zubereitungen enthaltenen Copolymerwachse z.B. Lippenstiften, Kajal- und Mascarastiften Stabilität auch bei höheren Temperaturen. Besonders bei Lippenstiften kann das hohe Ölbindevermögen und die hervorragende Dispergierwirkung genutzt werden. Als Binder kann das Copolymerwachs zusammen mit Lanolin, Paraffinöl, Isopropylstearat, Pigmenten und Parfüm für die Herstellung von Lidschatten, Augenbrauenstiften, Puder- und Rouge-Presslingen verwendet werden. Dabei werden die wasserabweisenden Eigenschaften, sowie die verdickende Wirkung dieser Copolymerwachse genutzt, um ein Verlaufen der Fettschminken zu unterdrücken.

Die oben beschriebenen Copolymerwachse eignen sich zur Herstellung kosmetischer, dermatologischer und pharmazeutischer Zubereitungen, besonders vorteilhaft zur Herstellung dekorativer kosmetischer Mittel, Sonnenschutzmittel, Deodorantien, Haarpflege- und Stylingmittel, Reinigungsmittel für die Haut und Peelings.

Eine bevorzugte Ausführungsform der erfindungsgemäßen Zubereitungen sind Emulsionen.

Bei den Emulsionen kann es sich beispielsweise um Wasser-in-Öl-Emulsionen, Öl-in-Wasser-Emulsionen, Mikroemulsionen, Nanoemulsionen und multiple Emulsionen handeln. Die Herstellung der Emulsionen kann in bekannter Weise, d.h. beispielsweise durch Kalt-, Heiß-, Heiß/Kalt- bzw. PIT-Emulgierung erfolgen. Eine besonders bevorzugte Ausführungsform sind selbstschäumende, schaumförmige, nachschäumende oder schäumbare Emulsionen und Mikroemulsionen.

Mit Hilfe von Emulgatoren lassen sich eine Vielzahl von Wachzubereitungen herstellen. Die Auswahl des Emulgators ermöglicht die Herstellung von nichtionogenen und ionogenen Wachsdispersionen.

Die erfindungsgemäß eingesetzten Copolymerwachse bewirken ein gutes und feinteiliges Aufnahmevermögen von Pigmenten und festen Wirkstoffen in der Ölphase und haben eine hautglättende und feuchtigkeitsspendende Wirkung. Die erfindungsgemäßen Zubereitungen zeichnen sich zudem durch eine besonders gute Haftung der kosmetischen Mittel auf der Haut aus und bilden hydrophobe Filme, die durch das sich bildende Hautfett kaum gelöst werden, so dass unerwünschte Farbverschiebungen der Pigmente bzw. eine Migrationen der Wirkstoffe unterbleiben.

Die erfindungsgemäßen Emulsionen enthalten mindestens
a) eines der oben beschriebenen Copolymerwachse,
b) eine Ölkomponente,
c) einen Emulgator und
d) optional weitere Wachse.

Die Ölkomponente kann vorteilhafterweise ausgewählt werden aus den Gruppen der Mineralöle, Mineralwachse, Öle, wie Triglyceride, Fette, Wachse und andere natürliche und synthetische Fettkörper, vorzugsweise Ester von Fettsäuren mit Alkoholen niedriger C-Zahl, z.B. mit Isopropanol, Propylenglycol oder Glycerin, oder Ester von Fettalkoholen mit Alkansäuren niedriger C-Zahl oder mit Fettsäuren oder aus der Gruppe der Alkylbenzoate.

Eine Klasse von erfindungsgemäß bevorzugten Ölen und Fetten sind die Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z.B. das Handelsprodukt Myritol® 318. Auch gehärtete Triglyceride sind erfindungsgemäß bevorzugt. Auch Öle tierischen Ursprungs, beispielweise Rindertalg, Perhydrosqualen, Lanolin können eingesetzt werden.

Eine weitere Klasse von erfindungsgemäß bevorzugten Ölen und Fetten sind die Benzoesäureester von linearen oder verzweigten C₈₋₂₂-Alkanolen, z.B. die Handelsprodukte Finsolv® SB (Isostearylbenzoat), Finsolv® TN (C₁₂-C₁₅-Alkylbenzoat) und Finsolv® EB (Ethylhexylbenzoat).

Eine weitere Klasse von erfindungsgemäß bevorzugten Ölen und Fetten sind die Dialkylether mit insgesamt 12 bis 36 Kohlenstoffatomen, insbesondere mit 12 bis 24 Kohlenstoffatomen, wie z.B. Di-n-octylether (Cetiol® OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether, Di-3-ethyldecylether, tert.-Butyl-n-octylether, iso-Pentyl-n-octylether und 2-Methyl-pentyl-n-octylether sowie Di-tert.-butylether und Di-iso-pentylether.

Eine weitere Klasse von erfindungsgemäß bevorzugten Ölen und Fetten sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, hydrierte Polyisobutylene, Docosane, Hexadecan, Isohexadecan, Paraffinöle, Paraffinwachse, Isoparaffinöle, z.B. die Handelsprodukte der Permethyl® -Serie, Squalan, Squalen, synthetische Kohlenwasserstoffe wie Polyisobuten und alicyclische Kohlenwasserstoffe, z.B. das Handelsprodukt 1,3-Di-(2-ethyl-hexyl)-cyclohexan (Cetiol® S), Ozokerit, Mikrowachse und Ceresin.

Ebenso in Betracht kommen verzweigte gesättigte oder ungesättigte Fettalkohole mit 6 - 30 Kohlenstoffatomen, z.B. Isostearylalkohol, sowie Guerbetalkohole.

Eine weitere Klasse von erfindungsgemäß bevorzugten Ölen und Fetten sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z.B. unter dem Handelsnamen Cosmacol® der EniChem, Augusta Industriale.

Eine weitere Klasse von erfindungsgemäß bevorzugten Ölen und Fetten sind Dicarbonsäureester von linearen oder verzweigten C₂-C₁₀-Alkanolen, wie Di-n-butyladipat (Cetiol® B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat.

Ebenso bevorzugte Öle und Fette sind symmetrische, unsymmetrische oder cyclische Ester der Kohlensäure mit Fettalkoholen, Glycerincarbonat oder Dicaprylylcarbonat (Cetiol® CC).

Eine weitere Klasse von erfindungsgemäß bevorzugten Ölen und Fetten sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

In einer weiteren bevorzugten Ausführungsform der Erfindung, wenn die Ölkomponente ein Silikonöl ist, liegen die erfindungsgemäßen Zubereitungen vorzugsweise in Form einer Wasser-in-Silikon-Emulsion vor und enthalten Wasser, Silikon, einen oder mehrere Emulgatoren und ein oder mehrere Copolymerwachse.

An Silikonölen bzw. -wachsen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare® Silicone 41 M65, SilCare® Silicone 41 M70, SilCare® Silicone 41 M80 (Clariant GmbH) erhältlichen Dimethicone, Stearyldimethylpolysiloxan, C₂₀-C₂₄-Alkyl-dimethylpolysiloxan, C₂₄-C₂₈-Alkyl-dimethylpolysiloxan, aber auch die unter SilCare® Silicone 41 M40, SilCare® Silicone 41 M50 (Clariant GmbH) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Erfindungsgemäße als Emulsionen vorliegende Zubereitungen enthalten einen oder mehrere Emulgatoren aus der Gruppe der nichtionischen, anionischen, kationischen oder amphoteren Emulgatoren.

Als nichtionogene Emulgatoren stehen vorzugsweise zur Verfügung Anlagerungsprodukte von 0 bis 30 Mol Ethylenoxid und/oder 0 bis 5 Mol Propylenoxid an lineare Fettalkohole mit 6 bis 30 C-Atomen, bevorzugt 10 bis 22 C-Atomen, und ganz besonders bevorzugt 14 bis 22 C-Atomen, gegebenenfalls hydroxyliert. Einsetzbar sind beispielsweise Octanol (Caprylalkohol), Octenol, Octadienol, Decanol (Caprinalkohol), Decenol, Decadienol, Dodecanol (Laurylalkohol), Dodecadienol, Ricinolalkohol, Myristylalkohol, Cetylalkohol, Stearylalkohol, Cetearylalkohol, Oleylalkohol, Linoleylalkohol, Linolenylalkohol, Arachidylalkohol, Behenylalkohol. Einsetzbar sind auch Fettalkoholschnitte, die durch Reduktion natürlich vorkommender Triglyceride, wie Rindertalg, Palmöl, Erdnussöl, Rüböl, Baumwollsaatöl, Sojaöl, Sonnenblumenöl und Leinöl erhalten werden oder die aus Umesterungsprodukten mit entsprechenden Alkoholen aus Fettsäureestern erzeugt werden und somit ein Gemisch von unterschiedlichen Fettalkoholen darstellen. Solche Substanzen sind beispielsweise unter der Bezeichnung Stenol® , z.B. Stenol® 1618, oder Lanette® , z.B. Lanette® O und Lanette® 22, oder Lorol® , z.B. Lorol® C18, im Handel erhältlich.

Geeignet sind auch Wollwachsfette.

Besonders bevorzugt sind Cetylalkohol, Stearylalkohol, Cetearylalkohol, Arachidylalkohol und Behenylalkohol.

Eine weitere Klasse von erfindungsgemäß bevorzugten Emulgatoren sind Anlagerungsprodukte von 0 bis 30 Mol Ethylenoxid und/oder 0 bis 5 Mol Propylenoxid an lineare und/oder verzweigte, gesättigte und/oder ungesättigte Fettsäuren mit 6 bis 30 C-Atomen, bevorzugt 10 bis 22 Kohlenstoffatomen. Zu nennen sind Isostearinsäure, wie die Handelsprodukte Emersol® 871 und Emersol® 875, Isopalmitinsäuren wie Edenor® IP95, sowie alle weiteren unter der Handelsbezeichnung Edenor® (Cognis) käuflichen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeosterinsäure, Arachinsäure, Gadoleinsäure, Behensäure, Erucasäure und Dimere von ungesättigten Fettsäuren, sowie deren technische Mischungen. Besonders bevorzugt sind Fettsäureschnitte aus Kokosöl oder Palmöl, insbesondere bevorzugt ist Stearinsäure.

Üblicherweise werden die Fettsäuren mit einem basischen Mittel, z.B. NaOH, neutralisiert und beispielsweise in Form ihrer Natrium-, Kalium-, Ammonium-, Calcium-, Magnesium- und Zink-Salze verwendet.

Eine weitere Klasse von bevorzugten Emulgatoren sind Ester von gewünschtenfalls alkylierten Zuckern mit C₆-C₃₀-Fettsäuren. Als Zucker können beliebige Mono- oder Oligosaccharide eingesetzt werden. Üblicherweise werden Monosaccharide mit 5 oder 6 Kohlenstoffatomen eingesetzt, beispielsweise Ribose, Xylose, Lyxose, Altose, Glucose, Fructose, Galactose, Arabinose, Altrose, Mannose, Gulose, Idose, Talose, sowie die Desoxyzucker Rhamnose und Fucose. Auch Zucker mit 4 Kohlenstoffatomen können eingesetzt werden, z.B. Erythrose und Threose. Erfindungsgemäß bevorzugte Oligosaccharide sind aus zwei bis 10 Monosaccharideinheiten zusammengesetzt, z.B. Sucrose (Saccharose), Lactose oder Trehalose. Bevorzugte Zuckerbausteine sind die Monosaccharide Glucose, Fructose, Galactose, Arabinose und das Disaccharid Sucrose. Glucose und Sucrose sind besonders bevorzugt. Die Zucker können partiell mit Methyl, Ethyl-, Propyl-, Isopropyl- oder Butylgruppen verethert sein, z.B. Methylglucosid, Ethylglucosid oder Butylglucosid. Zur Veresterung können alle C₆-C₃₀-Fettsäuren und deren Mischungen verwendet werden, die vorstehend genannt wurden. Geeignet sind prinzipiell einfach und mehrfach veresterte Zucker. Bevorzugt sind die Mono-, Sesqui- und Diester, beispielsweise Sucrosemonostearat, Sucrosedistearat, Sucrosemonococoat, Sucrosedicocoat, Methylglucosidmonostearat, Methylglucosidsesquistearat, Methylglucosidisostearat, Ethylglucosidmonolaurat, Ethylglucosiddilaurat, Ethylglucosidmonococoat, Ethylglucosiddicocoat und Butylglucosidmonococoat.

Eine weitere Klasse bevorzugter Emulgatoren sind C₈-C₂₂-Alkylmono- und -oligoglycoside, entsprechend der allgemeinen Formel RO-(Z)ₓ, wobei R für eine C₈-C₂₂-Alkylgruppe, Z für Zucker und x für die Anzahl der Zuckereinheiten stehen. Die erfindungsgemäß verwendbaren Alkylmono- und -oligoglycoside können lediglich einen bestimmten Alkylrest R enthalten. Besonders bevorzugt sind solche Alkylmono- und -oligoglycoside, bei denen R im wesentlichen aus C₈- und C₁₀₋Alkylgruppen, im wesentlichen aus C₁₂- und C₁₄-Alkylgruppen, im wesentlichen aus C₈- bis C₁₆-Alkylgruppen oder im wesentlichen aus C₁₂- bis C₁₆-Alkylgruppen besteht. Als Zuckerbaustein Z können beliebige Mono- oder Oligosaccharide, wie sie vorstehend genannt wurden, eingesetzt werden. Bevorzugte Zuckerbausteine sind Glucose, Fructose, Galactose, Arabinose und Sucrose, wobei Glucose besonders bevorzugt ist. Die erfindungsgemäß verwendbaren Alkylmono- und -oligoglycoside enthalten im Schnitt 1,1 - 5, bevorzugt 1,1 - 2,0 und besonders bevorzugt 1,1 - 1,8 Zuckereinheiten.

Auch die alkoxylierten Homologen der genannten Alkylmono- und -oligoglycoside können erfindungsgemäß bevorzugt eingesetzt werden. Diese Homologen können durchschnittlich bis zu 10 Ethylenoxid- und/oder Propylenoxideinheiten pro Alkylglycosideinheiten enthalten. Geeignete Alkylglycoside sind hierbei beispielsweise Cocoylglucosid, Decylglucosid, Laurylglucosid, Cetearylglucosid und Arachidylglucosid.

Besonders bevorzugt sind neben den genannten Alkylmono- und -oligoglucosiden auch die Gemische aus Alkylmono- und -oligoglucosiden und Fettalkoholen, z.B. die im Handel erhältlichen Produkte Montanov® 68 und Montanov® 202.

Eine weitere Klasse bevorzugter Emulgatoren sind die Partialester von Propylenglycol, Glycerin und Sorbitan mit C₈-C₂₂-Fettsäuren. Zur Veresterung können alle C₈-C₂₂-Fettsäuren und deren Mischungen verwendet werden, die vorstehend bereits genannt wurden. Besonders geeignete Beispiele sind Propylenglycolmonostearat, Glycerinmonolaurat, Glycerinmonostearat, Glycerindistearat, Glycerinmonooleat, Sorbitanmonolaurat, Sorbitandilaurat, Sorbitanmonostearat, Sorbitansesquistearat, Sorbitandistearat, Sorbitanmonoisostearat, Sorbitanmonooleat, Sorbitandioleat oder die Handelsprodukte Monomuls® 90-0, Monomuls® 90-L 12 und Cutina® MD. Diese Emulgatoren können durchschnittlich bis zu 10 Ethylenoxid- und/oder Propylenoxideinheiten pro Molekül enthalten.

Eine weitere bevorzugte Klasse an Emulgatoren sind Polyglycerine der Formel HO-CH₂-CHOH-CH₂[-O-CH₂-CHOH-CH₂]ₙ-O-CH₂-CHOH-CH₂OH mit n = 0-8 und deren Ester mit linearen und verzweigten C₈-C₂₂-Fettsäuren, die in der Alkylkette funktionelle Gruppen tragen können, bevorzugt Polyglyceryl-2-dipolyhydroxystearat (Handelsprodukt Dehymuls® PGPH) und Polyglyceryl-3-diisostearat (Handelsprodukt Lameform® TGI).

Eine weitere Klasse bevorzugter Emulgatoren sind Sterole (Sterine), insbesondere Cholesterol, Lanosterol, β-Sitosterol, Stigmasterol, Campesterol und Ergosterol sowie Mykosterole. Handelsübliche Sterol-Emulgatoren werden auf der Basis von Soja- oder Rapssterolen hergestellt. Erfindungsgemäß bevorzugt ist der Einsatz von Sterolen, die 5 - 10 Ethylenoxideinheiten pro Molekül enthalten. Geeignet sind z.B. die Handelsprodukte Generol® 122, Generol® 122 E 5, Generol® 122 E 10 und Generol® RE-10.

Ebenso bevorzugt einsetzbare Emulgatoren sind Phospholipide, vor allem die Phosphatidylcholine oder Lecithine. Phospholipide sind Phosphorsäurediester, seltener -monoester, von zumeist linearen gesättigten und ungesättigten C₈-C₂₂₋Fettsäuren. Bevorzugt ist Sojalecithin.

Eine weitere Klasse bevorzugter Emulgatoren sind die Veresterungsprodukte von Milchsäure oder Glykolsäure mit linearen oder verzweigten C₈-C₂₂-Fettsäuren sowie die Natrium-, Kalium-, Ammonium-, Calcium-, Magnesium- und Zink-Salze dieser Veresterungsprodukte.

Besonders bevorzugt sind Veresterungsprodukte der allgemeinen Formel (5) 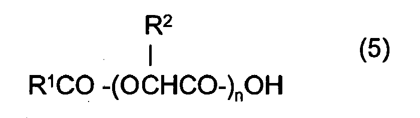 wobei R¹ einen linearen oder verzweigten gesättigten oder ungesättigten Alkylrest mit 5 bis 21 Kohlenstoffatomen und R² eine Methylgruppe oder ein Wasserstoffatom darstellen und n eine ganze Zahl von 1 - 4 ist.

Unter den Acylresten R¹CO- sind wiederum die Reste ausgewählt aus der Caproyl-, Capryloyl-, Caprinoyl-, Lauroyl-, Myristoyl-, Cetoyl-, Palmitoyl-, Stearoyl-, Isostearoyl- und der Oleyl-Gruppe bevorzugt. Besonders bevorzugt sind die Stearoyl- und die Isostearoyl-Gruppe. Der Rest R² ist vorzugsweise Methyl. Der Oligomerisierungsgrad n ist vorzugsweise 1 oder 2. Insbesondere bevorzugt ist die Verbindung Natriumstearoyl-2-lactylat.

Eine weitere Klasse bevorzugt eingesetzter Emulgatoren sind Phosphorsäuremono-, -di-, und -triester von gesättigten oder ungesättigten linearen oder verzweigten Fettalkoholen mit 8 bis 30 Kohlenstoffatomen und ihre Ethylenoxidaddukte mit 1 - 10 Ethylenoxid-Gruppen pro Molekül. Diese Alkyl- und Alkenylphosphate sind in der allgemeinen Formel (6) dargestellt 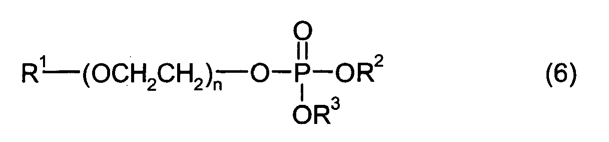 in der R¹ einen gesättigten oder ungesättigten, linearen oder verzweigten Kohlenwasserstoffrest mit 8 bis 30 Kohlenstoffatomen, R² und R³ unabhängig voneinander ein Wasserstoffatom, X oder einen Rest (CH₂CH₂O)ₙR¹, n Zahlen von 0 bis 10 und X ein Alkali- oder Erdalkalimetallkation oder ein Kation NR⁴R⁵R⁶R⁷, mit R⁴ bis R⁷ unabhängig voneinander stehend für einen C₁-C₄-Kohlenwasserstoffrest, darstellen.
Die erfindungsgemäß bevorzugten Alkyl- und Alkenylphosphate weisen als Gruppe R¹ Alkylreste mit 12 - 18 Kohlenstoffatomen auf, die gesättigt oder ungesättigt sowie linear oder verzweigt sein können. Diese Gruppen R¹ sind insbesondere Lauryl, Myristyl, Cetyl, Palmityl, Stearyl, Isostearyl und Oleyl. Bevorzugte Werte für n sind entweder 0 oder Werte von 1 - 10, bevorzugt 2 - 5, besonders bevorzugt 3 - 4 (Alkyl- oder Alkenyletherphosphate). Weiterhin bevorzugt ist die Verwendung von Estergemischen aus Mono-, Di- und Triestern, wobei der Anteil an Mono- und Diester gegenüber dem Triesteranteil überwiegt. Die Verwendung von reinen Triestern kann aber ebenfalls bevorzugt sein. Geeignete Handelsprodukte stammen z.B. aus der Hostaphat® -Serie (Clariant), z.B. Hostaphat® KW 340 D, Hostaphat® KO300 N, Hostaphat® KO380 und Hostaphat® KL 340.

Eine weitere Klasse von erfindungsgemäß bevorzugt eingesetzten Emulgatoren sind Acylglutamate der Formel (7) 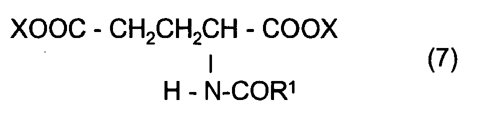 in der R¹CO für einen linearen oder verzweigten Acylrest mit 6 bis 22 Kohlenstoffatomen und 0, 1, 2 oder 3 Doppelbindungen und X für Wasserstoff, ein Alkali- oder Erdalkalimetallkation, für ein Ammonium, Alkylammonium, Alkanolammonium und/oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat.

Eine weitere Klasse von erfindungsgemäß bevorzugten Emulgatoren sind die Ester einer hydroxysubstituierten Di- oder Tricarbonsäure der allgemeinen Formel (8),

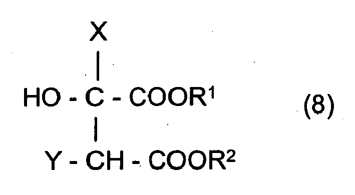

in der X H oder eine -CH₂COOR-Gruppe ist, Y H oder -OH ist unter der Bedingung, dass Y H ist, wenn X -CH₂COOR ist, R, R¹ und R² unabhängig voneinander ein Wasserstoffatom, ein Alkali- oder ein Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylsaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R, R¹ oder R² ein Rest Z ist.

Eine weitere Klasse von erfindungsgemäß bevorzugten Emulgatoren sind die Ester des Sulfobernsteinsäuresalzes der allgemeinen Formel (9)

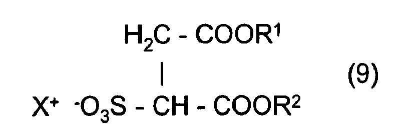

in der R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₈)-Hydroxyalkylpolyole mit 2 bis 16 Hydroxylresten ausgewählt ist, unter der Maßgabe, dass wenigstens eine der Gruppen R¹ oder R² ein Rest Z ist, und X⁺ ein Alkali- oder Erdalkalimetallkation, eine Ammoniumgruppe oder das Kation einer ammonium-organischen Base bedeutet.

Eine weitere Klasse von erfindungsgemäß bevorzugten Emulgatoren sind die Sulfobernsteinsäuremono- und -dialkylester mit 8 bis 24 C-Atomen in der Alkylgruppe und Sulfobernsteinsäuremonoalkylpolyoxyester mit 8 bis 24 C-Atomen in der Alkylgruppe und 1 bis 6 Ethoxygruppen und ihre Alkali-, Erdalkalimetall- oder Ammoniumsalze. Eine weitere Klasse von erfindungsgemäß bevorzugten Emulgatoren sind die Ester der Weinsäure und Zitronensäure mit Alkoholen, die Anlagerungsprodukte von etwa 2 bis 10 Molekülen Ethylenoxid und/oder Propylenoxid an Fettalkohole mit 8 bis 22 C-Atomen darstellen und ihre Alkali-, Erdalkalimetall- oder Ammoniumsalze.

Weitere erfindungsgemäß bevorzugte Emulgatoren sind Ethercarbonsäuren der Formel R-O-(CH₂-CH₂O)ₓ-CH₂-COOH, in der R eine lineare Alkylgruppe mit 8 bis 30 C-Atomen und x 0 oder 1 bis 10 ist, Acylsarcosinate mit einem linearen oder verzweigten Acylrest mit 6 bis 22 Kohlenstoffatomen und 0, 1, 2 oder 3 Doppelbindungen, Acyltaurate mit einem linearen oder verzweigten Acylrest mit 6 bis 22 Kohlenstoffatomen und 0, 1, 2 oder 3 Doppelbindungen sowie Acylisethionate mit einem linearen oder verzweigten Acylrest mit 6 bis 22 Kohlenstoffatomen und 0, 1, 2 oder 3 Doppelbindungen, sowie die Alkali-, Erdalkalimetall- oder Ammoniumsalze dieser Emulgatoren.

An amphoteren Emulgatoren stehen vorzugsweise zur Verfügung Alkylaminoalkylcarbonsäuren, Betaine, Sulfobetaine und Imidazolinderivate.

Vorzugsweise sind Fettalkoholethoxylate gewählt aus der Gruppe der ethoxylierten Stearylalkohole, Cetylalkohole, Cetylstearylalkohole, insbesondere
Polyethylenglykol(13)stearylether, Polyethylenglykol(14)stearylether,
Polyethylenglykol(15)stearylether, Polyethylenglykol(16)stearylether,
Polyethylenglykol(17)stearylether, Polyethylenglykol(18)stearylether,
Polyethylenglykol(1 9)stearylether, Polyethylenglykol(20)stearylether,
Polyethylenglykol(12)isostearylether, Polyethylenglykol(13)isostearylether,
Polyethylenglykol(14)isostearylether, Polyethylenglykol(15)isostearylether,
Polyethylenglykol(16)isostearylether, Polyethylenglykol(17)isostearylether,
Polyethylenglykol(18)isostearylether, Polyethylenglykol(19)isostearylether,
Polyethylenglykol(20)isostearylether, Polyethylenglykol(13)cetylether,
Polyethylenglykol(14)cetylether, Polyethylenglykol(15)cetylether,
Polyethylenglykol(16)cetylether, Polyethylenglykol(17)cetylether,
Polyethylenglykol(18)cetylether, Polyethylenglykol(19)cetylether,
Polyethylenglykol(20)cetylether, Polyethylenglykol(13)isocetylether,
Polyethylenglykol(14)isocetylether, Polyethylenglykol(15)isocetylether,
Polyethylenglykol(16)isocetylether, Polyethylenglykol(17)isocetylether,
Polyethylenglykol(18)isocetylether, Polyethylenglykol(19)isocetylether,
Polyethylenglykol(20)isocetylether, Polyethylenglykol(12)oleylether,
Polyethylenglykol(13)oleylether, Polyethylenglykol(14)oleylether,
Polyethylenglykol(15)oleylether, Polyethylenglykol(12)laurylether,
Polyethylenglykol(12)isolaurylether, Polyethylenglykol(13)cetylstearylether,
Polyethylenglykol(14)cetylstearylether, Polyethylenglykol(15)cetylstearylether,
Polyethylenglykol(16)cetylstearylether, Polyethylenglykol(17)cetylstearylether,
Polyethylenglykol(18)cetylstearylether, Polyethylenglykol(19)cetylstearylether,
Polyethylenglykol(20)cetylstearylether.

Vorzugsweise sind Fettsäureethoxylate gewählt aus der Gruppe der ethoxylierten Stearate, Isostearate und Oleate, insbesondere Polyethylenglykol(20)stearat,
Polyethylenglykol(21)stearat, Polyethylenglykol(22)stearat,
Polyethylenglykol(23)stearat, Polyethylenglykol(24)stearat,
Polyethylenglykol(25)stearat, Polyethylenglykol(12)isostearat,
Polyethylenglykol(13)isostearat, Polyethylenglykol(14)isostearat,
Polyethylenglykol(15)isostearat, Polyethylenglykol(16)isostearat,
Polyethylenglykol(17)isostearat, Polyethylenglykol(18)isostearat,
Polyethylenglykol(19)isostearat, Polyethylenglykol(20)isostearat,
Polyethylenglykol(21)isostearat, Polyethylenglykol(22)isostearat,
Polyethylenglykol(23)isostearat, Polyethylenglykol(24)isostearat,
Polyethylenglykol(25)isostearat, Polyethylenglykol(12)oleat,
Polyethylenglykol(13)oleat, Polyethylenglykol(14)oleat, Polyethylenglykol(15)oleat,
Polyethylenglykol(16)oleat, Polyethylenglykol(17)oleat, Polyethylenglykol(18)oleat,
Polyethylenglykol(19)oleat, Polyethylenglykol(20)oleat.

Als ethoxylierte Alkylethercarbonsäure oder deren Salze kann vorteilhafterweise das Natrium-laureth(11EO)-carboxylat verwendet werden.

Als Alkylethersulfat ist Lauryldiglycolethersulfat-Natriumsalz, als ethoxyliertes Cholesterinderivat Polyethylenglykol(30)Cholesterylether vorteilhaft. Ebenso bevorzugt ist Polyethylenglykol(25)Sojasterol.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat,
Polyethylenglykol(6)glycerylcaproat/caprinat, Polyethylenglykol(20)glyceryloleat,
Polyethylenglykol(20)glycerylisostearat und Polyethylenglykol(18)glyceryloleat/cocoat zu wählen.

Unter den Sorbitanestern eignen sich besonders Polyethylenglykol (20)sorbitanmonolaurat, Polyethylenglycol (20)sorbitanmonostearat, Polyethylenglycol (20)sorbitanmonoisostearat, Polyethylenglycol (20)sorbitanmonopalmitat und Polyethylenglykol (20)sorbitanmonooleat.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen,
Propylenglycolester gesättigter und/oder ungesättigter, verzweigter und/oder unverzweigter Alkancarbonsäuren einer Kettenlänge von 8 bis 24, insbesondere 12 bis 18 C-Atomen, sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.
Insbesondere vorteilhafte W/O-Emulgatoren sind Glycerylmonostearat, Glycerylmonoisostearat, Glycerylmonomyristat, Glycerylmonooleat, Glycerylmonolaurat, Glycerylmonocaprylat, Glycerylmonocaprinat, Diglycerylmonostearat, Diglycerylmonoisostearat, Propylenglykolmonostearat, Propylenglykolmonoisostearat, Propylenglykolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol oder Polyethylenglycol(2)stearylether.

In den erfindungsgemäßen Mitteln können Gemische von Verbindungen aus mehreren dieser Substanzklassen enthalten sein.

In einer weiteren bevorzugten Ausführungsform liegen die erfindungsgemäßen Zubereitungen in Form von Öl-in-Wasser-Emulsionen vor.

In einer besonders bevorzugten Ausführungsform liegen sie als kosmetische oder dermatologische Emulsionen vom Typ Öl-in-Wasser vor und enthalten bezogen auf das Gesamtgewicht der Zubereitung
a) bis zu 95 Gew.-%, vorzugsweise 60 bis 92 Gew.-%, besonders bevorzugt 70 bis 90 Gew.-%, insbesondere bevorzugt 75 bis 85 Gew.-% einer Wasserphase,
b) bis zu 40 Gew.-%, vorzugsweise 1 bis 40 Gew.-%, besonders bevorzugt 2 bis 25 Gew.-%, insbesondere bevorzugt 5 bis 20 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs.

In einer weiteren besonders bevorzugten Ausführungsform liegen die erfindungsgemäßen Zubereitungen als Gelcremes vom Typ Öl-in-Wasser, vorzugsweise als kosmetische oder dermatologische Gelcremes vom Typ Öl-in-Wasser, vor und enthalten bezogen auf das Gesamtgewicht der Zubereitungen
a) bis zu 95 Gew.-%, vorzugsweise 50 bis 95 Gew.-%, besonders bevorzugt 70 bis 90 Gew.-%, insbesondere bevorzugt 75 bis 85 Gew.-% einer Wasserphase,
b) bis zu 30 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) bis zu 5 Gew.-%, vorzugsweise 0,5 bis 5 Gew.-%, besonders bevorzugt 0,2 bis 4 Gew.-%, insbesondere bevorzugt 0,5 bis 3 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs.

In einer weiteren bevorzugten Ausführungsform liegen die erfindungsgemäßen Zubereitungen in Form von Wasser-in-Öl-Emulsionen vor.

In einer besonders bevorzugten Ausführungsform liegen sie als kosmetische oder dermatologische Emulsionen vom Typ Wasser-in-Öl vor und enthalten bezogen auf das Gesamtgewicht der Zubereitungen
a) bis zu 95 Gew.-%, vorzugsweise 40 bis 95 Gew.-%, besonders bevorzugt 50 bis 90 Gew.-%, insbesondere bevorzugt 60 bis 85 Gew.-% einer Wasserphase,
b) bis zu bis zu 60 Gew.-%, vorzugsweise 2 bis 60 Gew.-%, besonders bevorzugt 5 bis 40 Gew.-%, insbesondere bevorzugt 10 bis 30 Gew.-% einer Ölphase,
c) bis zu 20 Gew.-%, vorzugsweise 0,5 bis 20 Gew.-%, besonders bevorzugt 1 bis 15 Gew.-%, insbesondere bevorzugt 4 bis 12 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs.

In einer weiteren besonders bevorzugten Ausführungsform liegen die erfindungsgemäßen Zubereitungen in Form von Wasser-in-Silikon-Emulsionen vor.

Hierunter wiederum bevorzugt sind Wasser-in-Silikon-Emulsionen, vorzugsweise kosmetische oder dermatologische Wasser-in-Silikon-Emulsionen, die bezogen auf das Gesamtgewicht der Zubereitungen
a) bis zu 90 Gew.-%, vorzugsweise 20 bis 90 Gew.-%, besonders bevorzugt 40 bis 85 Gew.-%, insbesondere bevorzugt 60 bis 80 Gew.-% einer Wasserphase,
b) bis 80 Gew.-%, vorzugsweise 10 bis 70 Gew.-%, besonders bevorzugt 20 bis 60 Gew.-%, insbesondere bevorzugt 30 bis 50 Gew.-% an Silikonöl,
c) 0,5 bis 20 Gew.-%, vorzugsweise 1 bis 15 Gew.-%, besonders bevorzugt 3 bis 10 Gew.-% eines oder mehrerer Emulgatoren und
d) 0,01 bis 5 Gew.-%, vorzugsweise 0,05 bis 3 Gew.-%, besonders bevorzugt 0,1 bis 2 Gew.-% an Copolymerwachs
enthalten.

In einer weiteren bevorzugten Ausführungsform werden die in den erfindungsgemäßen Zubereitungen enthaltenen Copolymerwachse in Form von mikronisierten Wachsen eingesetzt. Hierbei werden sie vorzugsweise in Teilchengrößen von 4 bis 45 µm und besonders bevorzugt in Teilchengrößen von 4 bis 20µm eingesetzt.

Mikronisierte Copolymerwachse aus feinsten, runden Partikeln mit enger Korngrößenverteilung können aus einer Wachsschmelze in einem Sprühverfahren hergestellt werden.

Die mikronisierten Copolymerwachse lassen sich leichter dispergieren, bewirken bessere Gleiteigenschaften der Zubereitungen und verbessern das Hautgefühl und die Verteilbarkeit der Mittel auf der Haut und auf den Haaren. Besonders vorteilhaft können die oben beschriebenen Copolymerwachse in Peelings zum Reinigen und Pflegen der Haut eingearbeitet werden

Eine weitere bevorzugte Ausführungsform der Erfindung sind Wachsdispersionen, enthaltend
a) ein Trägermaterial, vorzugsweise ein oder mehrere Ölkomponenten und/oder Lösungsmittel,
b) ein oder mehrere Emulgatoren und
c) ein oder mehrere der oben beschriebenen Copolymerwachse und gegebenenfalls ein oder mehrere weitere Wachse.

Wachsdispersionen, enthaltend
a) ein Trägermaterial, vorzugsweise ein oder mehrere Ölkomponenten und/oder Lösungsmittel,
b) ein oder mehrere Emulgatoren und
c) ein oder mehrere der oben beschriebenen Copolymerwachse und gegebenenfalls ein oder mehrere weitere Wachse
und worin der Wachsgehalt von 20 bis 45 Gew.-% beträgt, können auch als fließfähige Zubereitung zur Einarbeitung in kosmetische, pharmazeutische und dermatologische Mittel dienen.

Die erfindungsgemäßen Zubereitungen können neben den Copolymerwachsen bzw. mikronisierten Copolymerwachsen weitere Wachse, wie z.B. Polyethylenwachse, oxidierte Polyethylenwachse, Amidwachse, Carnaubawachse, Montanwachse, Paraffinwachs, Fischer-Tropsch-Wachse oder Polyvinylwachse, gegebenenfalls in Kombination mit hydrophilen Wachsen wie z.B. Cetylstearylalkohol, enthalten.

In einer weiteren bevorzugten Ausführungsform handelt es sich bei den erfindungsgemäßen Zubereitungen um dekorative Mittel.

Eine weitere bevorzugte erfindungsgemäße Ausführungsform sind kosmetische und dermatologische Stifte, beispielsweise Lippenstifte, Sonnencremestifte, Antiakne-Stifte, Augenbrauenstifte, Abdeckstifte und Deo-Stifte, enthaltend
a) eine Lipidphase aus mindestens einer Ölkomponente und mindestens einem Copolymerwachs, wie oben beschrieben,
b) optional in der Lipidphase lösliche oder dispergierbare Substanzen,
c) eine wässrige Phase,
d) optional in Wasser lösliche oder dispergierbare Substanzen,
e) optional eine oder mehrere Wirksubstanzen und
f) mindestens einen W/O-Emulgator,
wobei der Anteil der wässrigen Phase, bezogen auf die fertige Zubereitung, 30 bis 80 Gew.-% betragen kann.

In einer weiteren bevorzugten Ausführungsform, insbesondere wenn es sich um dekorative Mittel handelt, enthalten die erfindungsgemäßen kosmetischen und dermatologischen Zubereitungen ein oder mehrere Farbmittel, vorzugswese ausgewählt aus Farblacken, Toner und Pigmenten. Hierbei liegen sie vorzugsweise in Form von Pudern, Preßlingen, Pasten, Cremes oder Sticks vor.

In einer besonders bevorzugten Ausführungsform liegen die erfindungsgemäßen Zubereitungen in Form von Suspensionen vor und enthalten bezogen auf das Gesamtgewicht der Zubereitung
a) 0,1 bis 10 Gew.-%, vorzugsweise 0,2 bis 6 Gew.-% und besonders bevorzugt 0,3 bis 5 Gew.-% an Copolymerwachs und
b) 0,1 - 30 Gew.-%, vorzugsweise 0,5 bis 15 Gew.-% und besonders bevorzugt 1,0 bis 10 Gew.-% an festen Partikeln, insbesondere ausgewählt aus der Gruppe der Farbstoffe, farbgebenden Pigmente, Effekt- und Lichtschutzpigmente, Adsorbentien und Abrassivkomponenten.

In einer weiteren besonders bevorzugten Ausführungsform liegen die erfindungsgemäßen Zubereitungen als Lidschatten auf Gelbasis vor und enthalten bezogen auf das Gesamtgewicht der Zubereitung
a) 0,1 bis 10 Gew.-%, vorzugsweise 0,2 bis 6 Gew.-% und besonders bevorzugt 0,3 bis 5 Gew.-% an Copolymerwachs und
b) 0,1 bis 30 Gew.-%, vorzugsweise 0,5 bis 15 Gew.-% und besonders bevorzugt 1,0 bis 10 Gew.-% an Farbstoffen und/oder farbgebenden Pigmenten.

Die erfindungsgemäßen Mittel können feste anorganische und organische Partikel enthalten. Für dekorative Kosmetika werden farbige und auch farblose Pigmente eingesetzt. Einige der nachfolgend genannten Pigmente dienen auch als UV-Absorber bzw. Lichtschutzpigmente.

Die Farbstoffe- und -pigmente, sowohl organische, als auch anorganische Farbstoffe können aus der entsprechenden Positivliste der Kosmetikverordnung bzw. der EG-Liste kosmetischer Färbemittel ausgewählt werden.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-sulfosäure | 13015 | gelb |
| 2,4-Dihydroxy-azobenzol-4'-sulfosäure | 14270 | orange |
| 2-(2,4-Dimethylphenylazo-5-sulfosäurse)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-Chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1'-naphthylazo]-1-hydroxy-7-aminoaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)- | 42090 | blau |
| methylen-(N-ethyl-N-p-sulfo-benzyl)-cyclohexadienimin | | |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsinimmonium | 44045 | blau |
| 2-Hydroxy-3,6-disulfo-4,4'-bis-dimethylaminonaphtofuchsinimmonium. | 44090 | grün |
| Acid red | 45100 | rot |
| 3,-(2'-Methylphenylamino)-6-(2'-methyl-.4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluprescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium-, und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z.B. Paprikaextrakte, β-Carotin und Cochenille.

Vorteilhaft eingesetzt werden auch Perlglanzpigmente, z.B. Fischsilber (Guanin/ Hypoxanthin-Mischkristalle aus Fischschuppen) und Perlmutt (vermahlene Muschelschalen), monokristalline Perlglanzpigmente wie z.B. Bismuthoxychlorid (BiOCl), Schicht-Substrat Pigmente, z.B. Glimmer/Metalloxid, silberweiße Perlglanzpigmente aus TiO₂, Interferenpigmente (TiO₂, unterschiedliche Schichtdicke), Farbglanzpigmente (Fe₂O₃) und Kombinationspigmente (TiO₂/Fe₂O₃, TiO₂/Cr₂O₃, TiO₂/Berliner Blau, TiO₂/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z.B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z.B. Bismutoxychlorid (BiOCI), sowie die Oxide von beispielsweise Titan, insbesondere die TiO₂-Modifikationen Anatas und Rutil, Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z.B. von 2 - 50 µm, 5 - 25 µm, 5 - 40 µm, 5 - 60 µm, 5 - 95 µm, 5-100 µm, 10 - 60 µm, 10 - 100 µm, 10 - 125 µm, 20 - 100 µm, 20 - 150 µm, sowie < 15 µm. Eine breitere Teilchengrößenverteilung z.B. von 20 - 150 µm, ruft glitzernde Effekte hervor, während eine engere Teilchengrößenverteilung von < 15 µm für eine gleichmäßige seidige Erscheinung sorgt.

Die erfindungsgemäßen Zubereitungen enthalten Effektpigmente vorzugsweise in Mengen von 0,1 - 20 Gew.-%, besonders bevorzugt 0,5 - 10 Gew.-% und insbesondere bevorzugt 1 - 5 Gew.-%, jeweils bezogen auf das Gesamtgewicht der Zubereitung.

Bei den bevorzugten anorganischen Lichtschutzpigmenten handelt es sich um feindisperse oder kolloiddisperse Metalloxide und Metallsalze, beispielsweise Titanoxid, Zinkoxid, Eisenoxid, Aluminiumoxid, Ceroxid, Zirkoniumoxid, Silikate (Talk) und Bariumsulfat. Die Partikel sollten dabei einen mittleren Durchmesser von weniger als 100 nm, vorzugsweise zwischen 5 und 50 nm und besonders bevorzugt zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyocytylsilane oder Simethicone in Frage. Besonders bevorzugt sind Titandioxid und Zinkoxid.

Die bevorzugten anorganischen Partikelsubstanzen sind hydrophil oder amphiphil. Vorteilhafterweise können sie oberflächlich beschichtet, insbesondere oberflächlich wasserabweisend behandelt sein. Beispiele hiefür sind mit Aluminiumstearat beschichtete Titanoxid-Pigmente, mit Dimethylpolysiloxan (Dimethicone) beschichtetes Zinkoxid, mit Dimethicone beschichtetes Bornitrid und mit einem Gemisch aus Dimethylpolysiloxan und Silicagel und Aluminiumoxidhydrat beschichtetes Titanoxid, mit Octylsilanol beschichtetes Titanoxid oder sphärische Polyalkylsesquioxan-Partikel.

Bei organischen Lichtschutzpigmenten handelt es sich um bei Raumtemperatur kristallin vorliegende Substanzen, die in der Lage sind, ultraviolette Strahlen zu absorbieren und die aufgenommene Energie in Form längerwelligerer Strahlung, z.B. Wärme, wieder abzugeben. Man unterscheidet UVA-Filter und UVB-Filter. Die UVA- und UVB-Filter können sowohl einzeln als auch in Mischungen eingesetzt werden. Die erfindungsgemäß geeigneten organischen UV-Filter sind ausgewählt aus den bei Raumtemperatur festen Derivaten von Dibenzoylmethan, Zimtsäureestern, Diphenylsäureestern, Benzophenon, Campher, p-Aminobenzoesäureestern, o-Aminobenzoesäureestern, Salicylsäureestern, Benzimidazolen, 1,3,5-Triazinen, monomeren und oligomeren 4,4-Diarylbutadiencarbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein. Erfindungsgemäß besonders bevorzugte öllösliche UV-Filter sind (1-(4-tert-Butylphenyl)-3-(4'-methoxyphenyl)propan-1,3-dion, 1-Phenyl-3-(4'-Isopropylphenyl)-propan-1,3-dion, 3-(4'-Methylbenzyliden)-D,L-campher, 4-(Dimethylamino)-benzoesäure-2-ethylhexylester, 4-(Dimethylamino)-benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-octylhexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino(p-carbo-2'-ethyl-1'-hexyloxy)-1,3,5-triazin (Octyl Triazone) und Dioctyl Butamido Triazone sowie beliebige Mischungen der genannten Komponenten.

Bevorzugte wasserlösliche UV-Filter sind 2-Phenylbenzimidazol-5-sulfonsäure und deren Alkali, Erdalkali, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z.B. 4-(2-oxo-3-bornylidenmethyl)benzolsulfonsäure und 2-Methyl-5-(2-oxo-3-bornyliden)sulfonsäure und deren Salze.

In den erfindungsgemäßen Zubereitungen sind die anorganischen und organischen Lichtschutzpigmente in Mengen von vorzugsweise 0,1 bis 30 Gew.-%, besonders bevorzugt 1 bis 20 Gew.-% und insbesondere bevorzugt 2 bis 15 Gew.-%, jeweils bezogen auf das Gesamtgewicht der Zubereitung, enthalten.

Die erfindungsgemäßen Zubereitungen können teilchenförmige anorganische oder organische Adsorbentien mit mittleren Partikeldurchmessern von 1 - 100 µm, enthalten. Die Adsorbentien sind ausgewählt aus pyrogenen Kieselsäuren, z.B. den Aerosol-Typen, Fällungskieselsäuren, Kieselgelen, Siliciumdioxid, Tonen, z.B. Bentonite oder Kaolin, Magnesiumaluminiumsilikaten, z.B. Talkum und Bornitrid, gegebenenfalls modifizierte Stärken und Stärkederivate, Cellulosepulvern, Lactoglobulinderivaten, Polymerpulvern aus Polyolefinen, Polycarbonaten, Polyurethanen, Polyamiden, Polyestern, Polystyrolen, Polyacrylaten, (Meth)acrylat-oder (Meth)acrylat-Vinyliden-Copolymeren, die vernetzt sein können, Teflon oder Siliconen, sowie Mischungen der genannten Substanzen.

Die erfindungsgemäßen Zubereitungen können Abrasivkomponenten enthalten, beispielsweise gemahlene Pflanzenteile wie Mandelkleie oder Weizenkleie, kristalline Cellulose, gehärtetes Jojobaöl, Polymerkügelchen, bevorzugt aus Polyethylen oder Polyamid-11, mit mittleren Durchmessern von 90-600 µm, und aus wirkstoffhaltigen Mikro- oder Millikapseln, die petrochemische Polymere (z.B. aus Polyamid wie Nylon-11) und/oder Biopolymere wie Gelatine, Pektin, Pflanzlichen Gummen, Alginaten und Carrageenan enthalten. Bevorzugt eingesetzt werden Mandelkleie, Weizenkleie, gehärtetes Jojobaöl und Polyethylenkügelchen.

In einer bevorzugten Ausführungsform der Erfindung handelt es sich um make-up, Augen-make-up, Mascara, eyeliner und Rouge, gekennzeichnet durch besondere Wasserfestigkeit, Farbbrillianz, Perlglanzeffekt, gute Hautsensorik und gute Verteilbarkeit der Mittel auf der Haut.

In einer weiteren bevorzugten Ausführungsform der Erfindung handelt es sich um Nagellack mit ausgezeichneten Glanzeffekten.

In einer weiteren bevorzugten Ausführungsform enthalten die erfindungsgemäßen Zubereitungen eine oder mehrere UV-Lichtschutzfilter. Bei diesen erfindungsgemäßen Zubereitungen handelt es sich vorzugsweise um Sonnenschutzmittel. Die Sonnenschutzmittel liegen vorzugsweise in Form von Sprays, Sticks, Pasten, Gelen oder Lotionen vor.

Als UV-Filter kommen vorzugsweise in Betracht 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäureisoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-aminocarbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat, N,N,N-Trimethyl-4-(2-oxoborn-3-ylidenemethyl) anilium methyl sulphate, Homosalate (INN) Oxybenzone (INN) 2-Phenylbenzimidazole-5-sulfonsäure und ihre Na, K, und Triethanolaminsalze, alpha-(2-Oxoborn-3-ylidene) toluol-4-sulfonsäure und ihre Salze, Octyl methoxyzimtsäure, Isopentyl-4-methoxyzimtsäure, Isoamyl p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometriazole Trisiloxane) benzoesäure, 4,4-((6-(((1,1-dimethylethyl)amino)carbonyl)phenyl)amino)-1,3,5-triazine-2,4-diyl)diimino)bis-,bis(2-ethylhexyl)ester) benzoesäure, 4,4-((6-(((1,1-dimethylethyl)amino)-carbonyl)phenyl)amino)-1,3,5-triazine-2,4-diyl)diimino)bis-,bis(2-ethylhexyl)ester) 3-(4'-Methylbenzylidene)-d-1 camphor (4-Methylbenzylidene Camphor) 3-Benzylidene camphor 2-Ethylhexyl salicylat (Octyl Salicylat) 4-Dimethylaminobenzoat von ethyl-2-hexyl (octyl dimethyl PABA), 2-Hydroxy-4-methoxybenzophenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat Di-p-methoxy zimtsäure, p-Aminobenzoesäure und Ester, 4-tert-Butyl-4'-methoxydibenzoylmethan, 4-(2-β-glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-dihydroxy-4,4'-dimethoxy-5,5'-disulfobenzophenon, Dihydroxybenzophenon, 1-3,4-Dimethoxyphenyl)-4,4-dimethyl-1,3-pentanedion, 2-Ethylhexyl dimethoxybenzylidene dioxoimidazolidin propionat, Tetrahydroxybenzophenone, Terephthalyldendicamphorsulfonsäure, 2,4,6-tris[4-2-ethylhexyloxycarbonyl)anilino]-1,3,5-triazin, Methyl bis(trimethylsiloxy)silyl isopentyltrimethoxy-zimtsäure, Amylp-dimethylamino benzoat, 2-Ethylhexyl-p-dimethylamino benzoat, Isopropyl-p-methoxyzimtsäure/Diisopropylzimtsäureester, 2-Ethylhexyl p-methoxyzimtsäure, 2-Hydroxy-4-methoxybenzophenon, 2-Hydroxy-4-methoxybenzophenon-5-sulfonsäure und das Trihydrat, 2-Hydroxy-4-methoxybenzophenon-5-sulfonat, Na-Salz, Phenyl benzimidazolsulfonsäure.
Die erfindungsgemäßen Zubereitungen enthalten UV-Lichtschutzfilter in den Mengen von vorzugsweise 0,1 bis 10 Gew.-%, besonders bevorzugt 0,5 bis 8 Gew.-% und insbesondere bevorzugt 1 bis 5 Gew.-%, bezogen auf die fertigen Zubereitungen.

In einer weiteren bevorzugten Ausführungsform enthalten die erfindungsgemäßen Zubereitungen, beispielsweise die Sonnenschutzmittel, ein oder mehrere Antioxidantien.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. (α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfon, Penta-, Hexa-, Heptathioninsulfoximin) in sehr geringen verträglichen Dosierungen, ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den erfindungsgemäßen Zubereitungen beträgt vorzugsweise 0,001 bis 30 Gew.-%, besonders bevorzugt 0,05 bis 20 Gew.-% und insbesondere 1 bis 10 Gew.-%, bezogen auf das Gesamtgewicht der Zubereitungen.

Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der Zubereitung zu wählen.

Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Zubereitung, zu wählen.

In einer besonders bevorzugten Ausführungsform der Erfindung enthalten die kosmetischen oder pharmazeutischen Zubereitungen Antioxidantien ausgewählt aus Superoxid-Dismutase, Tocopherol (Vitamin E) und Ascorbinsäure (Vitamin C).

In einer weiteren bevorzugten Ausführungsform handelt es sich bei den erfindungsgemäßen Zubereitungen um Deodorantien und Antiperspirantien, die ein oder mehrere Substanzen ausgewählt aus antimikrobiell wirkenden Substanzen, Adstringentien und deodorierenden Stoffen enthalten. Diese Zubereitungen liegen vorzugsweise in Form von Sprays, Sticks, Pasten, Gelen oder Lotionen vor. Bevorzugt geeignet als antimikrobielle Wirkstoffe sind Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol und Kombinationen dieser Wirksubstanzen.

Die erfindungsgemäßen Zubereitungen enthalten die antimikrobiellen Wirkstoffe bevorzugt in Mengen bis 50 Gew.-%, besonders bevorzugt in Mengen von 0,01 bis 10 Gew.-% und insbesondere bevorzugt in Mengen von 0,1 bis 10 Gew.-%, bezogen auf die fertigen Zubereitungen.

Bevorzugte Adstringentien sind Oxide, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink.

Die erfindungsgemäßen Zubereitungen enthalten die adstringenden Wirkstoffe bevorzugt in Mengen von 0 bis 50 Gew.-%, besonders bevorzugt in Mengen von 0,01 bis 10 Gew.-% und insbesondere bevorzugt in Mengen von 0,1 bis 10 Gew.-%, bezogen auf die fertigen Zubereitungen.

Als deodorierende Stoffe bevorzugt sind Allantoin und Bisabolol. Diese werden vorzugsweise in Mengen von 0,0001 bis 10 Gew.-% eingesetzt.

In einer weiteren bevorzugten Ausführungsform handelt es sich bei den erfindungsgemäßen Zubereitungen um Peelings. Diese liegen vorzugsweise in Form von Peeling-Cremes oder -gelen zum Reinigen und Glätten der Haut vor.

Die erfindungsgemäßen Zubereitungen können als weitere Hilfs- und Zusatzstoffe andere pulverförmige Stoffe, Füllkörper, kationische Polymere, Filmbildner, Verdickungs- und Dispergiermittel, Überfettungsmittel, feuchtigkeitsspendende Mittel, Stabilisatoren, biogene Wirkstoffe, Glycerin, Konservierungsmittel, Perlglanzmittel, Duftstoffe, Lösungsmittel, Trübungsmittel, weitere Wachse, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, Erfrischungsmittel, beispielsweise Methylacetat, Stoffe mit keratolytischer und keratoplastischer Wirkung, Enzyme und Trägersubstanzen enthalten.

Des weiteren können als Füllkörper SiO₂, Silica, ZnO, Kaolin, mit SiO₂ modifiziertem Kaolin, Polytetrafluorethylen, Nylon, Talkum, Glimmer, Polymethylmethacrylat, Polyethylen, Polyether, Polycarbonate, Polyvinylchlorid, Polystyrol, Polyamide, Polyurethane, Polyacrylate, natürliche Polymere, Seidenpulver, mikrokristalline Cellulose, natürliche organische Verbindungen wie verkapselte oder unverkapselte Getreidestärke und Gemische davon eingesetzt werden.

An kationischen Polymeren stehen die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guar-hydroxypropyltriammoniumchloride, sowie Calciumalginat und Ammoniumalginat zur Verfügung. Ebenso geeignet sind kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan.

Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane, Polyethersiloxan-Copolymere, wie in US 5,104,645 und den darin zitierten Schriften beschrieben, die bei Raumtemperatur sowohl flüssig als auch harzförmig vorliegen können.

Geeignete Filmbildner sind, je nach Anwendungszweck Salze der Phenylbenzimidazolsulfonsäure, wasserlösliche Polyurethane, beispielsweise C₁₀-Polycarbamyl-Polyglycerolester, aber auch Polyvinylalkohol, Polyvinylpyrrolidon, sowie Copolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carbocyvinyl-Polymere, wie Carbomere und deren Salze, Polysaccharide, beispielsweise Polydextrose und Glucan.

Als Überfettungsmittel können Substanzen wie beispielsweise Lanilin, Lecithin, polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Als feuchtigkeitsspendende Substanz stehen beispielsweise Isopropylpalmitat, Glycerin und/oder Sorbitol zu Verfügung die bevorzugt in den Mengen 0,1 bis 50 Gew.-% eingesetzt werden.

Als biogene Wirkstoffe können beispielsweise Pflanzenextrakte, beispielsweise Aloe Vera und Vitaminkomplexe, Bisabolol® , Allantoin® , Phytantriol® , Panthenol® , AHA-Säuren, Lokalanästhetika, Antibiotika, Antiphlogistika, Antiallergica, Corticosteoide, Sebostatika, Phanthenol, Allantoin und Proteine eingesetzt werden.
Als Konservierungsmittel eignen sich beispielsweise Phenoxyethanol, Diazolidinylharnstoff, Parabene, Pentandiol, butyliertes Hydroxytoluol, butyliertes Hydroxyanisol oder Sorbinsäure. Sie werden vorzugsweise in den Mengen von 0,001 bis 5 Gew.-%, besonders bevorzugt von 0,01 bis 3 Gew.-% und insbesondere bevorzugt von 0,1 bis 2 Gew.-%, bezogen auf die fertigen Zubereitungen eingesetzt.

Als Farbstoffe können die für kosmetische und pharmazeutische Zwecke geeigneten und zugelassenen Substanzen verwendet werden.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z.B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykoldistearate mit durchschnittlich 3 Glykoleinheiten.

Sofern die erfindungsgemäßen Zubereitungen perlglanzgebende Verbindungen enthalten, sind diese bevorzugt in einer Menge von 0,1 bis 15 Gew.-% und besonders bevorzugt in einer Menge von 1 bis 10 Gew.-% in den erfindungsgemäßen Zubereitungen enthalten.

Als antifungizide Wirkstoffe eignen sich bevorzugt Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Zn-Pyrethion und Octopyrox.

Als Verdickungs- und Dispergiermittel besonders geeignet sind Ethylenglykolester von Fettsäuren mit 14 bis 22, besonders bevorzugt 16 bis 22, Kohlenstoffatomen, insbesondere Mono- und Di-ethylenglykolstearat. Ebenfalls bevorzugt geeignet sind Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl-(C₁₂-C₂₂)-amidobenzoesäure und deren lösliche Salze, N,N-Dihydrocarbyl-(C₁₆-C₁₈)-amidobenzoesäure und deren lösliche Salze und N,N-di(C₁₆-C₁₈)-amidobenzoesäure und deren Derivate. Weiterhin besonders geeignet sind Polyacrylate und Carbomere, insbesondere solche wasserlöslicher oder wasserquellbarer Copolymerisate auf Basis von Acrylamidoalkylsulfonsäuren und N-Vinylcarbonsäureamiden.

Zur Erhöhung der Farbintensität können die erfindungsgemäßen Zubereitungen die in kosmetischen Systemen üblichen Carrier enthalten, insbesondere Benzylalkohol, Vanillin (4-Hydroxy-3-methoxy-benzaldehyd), Isovanillin, p-Hydroxyanisol, 3-Hydroxy-4-methoxy-benzaldehyd, 2-Phenoxyethanol, Salicylaldehyd, 3,5-Dihydroxybenzaldehyd, 3,4-Dihydroxybenzaldehyd, 4-Hydroxyphenylacetamid, p-Hydroxybenzoesäure-methylester, p-Hydroxybenzaldehyd, m-Kresol, Hydrochinonmonomethylether, o-Fluorphenol, m-Fluorphenol, p-Fluorphenol, 2-(2'-Hydroxyphenoxy)-ethanol, 3,4-Methylendioxyphenol, Resorcinmonomethylether, 3,4-Dimethoxyphenol, 3-Trifluormethylphenol, Resorcinmonoacetat, Ethylvanillin, 2-Thiophenethanol, Milchsäurebutylester und Glykolsäurebutylester. Besonders vorteilhaft mit synergistischer Wirkung sind erfindungsgemäße Zubereitungen enthaltend Phenoxyethanol und/oder Benzylalkohol.

Als Solubilisatoren eignen sich prinzipiell alle ein- oder mehrwertigen Alkohole und ethoxylierten Alkohole. Bevorzugt werden Alkohole mit 1 bis 4 Kohlenstoffatomen, wie z.B. Ethanol, Propanol, Isopropanol, n-Butanol und Iso-Butanol, Glycerin und deren Mischungen eingesetzt. Weiterhin bevorzugt sind Polyethylenglykole mit einer relativen Molekülmasse unter 2000. Insbesondere bevorzugt sind Polyethylenglykole mit einer relativen Molekülmasse zwischen 200 und 600 in Mengen bis zu 45 Gew.-% und Polyethylenglykole mit einer relativen Molekülmasse zwischen 400 und 600 in Mengen von 0,5 bis 15 Gew.-%. Weitere geeignete Lösungsmittel sind beispielsweise Triacetin (Glycerintriacetat) und 1-Methoxy-2-propanol.

Als Stabilisatoren können Metallsalze von Fettsäuren, wie z.B. Magnesium-, Aluminium- und/oder Zinkstearat eingesetzt werden.

Die erfindungsgemäßen Zubereitungen können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.
Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Hydroxycitronellal, Lilial und Bourgeonal, zu den Ketonen z.B. die lonone, alpha-Isomethylionon und Methylcedrylketon, zu den Alkoholen Anethol, Citronellol, Eugenol, Geranion, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Weitere Zusatzstoffe können Siliconverbindungen sein, vorzugsweise Dimethylpolysiloxan, Methyl-phenylpolysiloxane, cyclische Silicone sowie amino-, fettsäure-, alkohol-, polyether-, epoyx-, fluor- und/oder alkylmodifizierte Siliconverbindungen, beispielsweise Alkylsilicone SilCare® Silicone 41M10, SilCare® Silicone 41 M15, SilCace® Silicone 41 M20, SilCare® Silicone 41 M30 (Clariant), Alkyltrimethicone SilCare® 31 M30, SilCare® 31 M40, SilCare® 31 M 50, SilCare® 31 M 60 (Clariant), Phenyltrimethicone SilCare® 15M30, SilCare® 15M40, SilCare® 15M50, SilCare® 15M60 (Clariant), Polyalkylarylsiloxane und Polyethersiloxan-Copolymere.

Die erfindungsgemäßen Zubereitungen können die oben genannten Siliconverbindungen vorzugsweise in den Gewichtsmengen von 0,1 bis 20 Gew.-%, besonders bevorzugt von 0,2 bis 15 Gew.-% und insbesondere bevorzugt von 0,5 bis 10 Gew.-% bezogen auf die fertigen Zubereitungen enthalten.

Die Zubereitungen besitzen üblicherweise einen pH Wert im Bereich von 2 bis 12 und bevorzugt in einem Bereich von 3 bis 8.

Eine herausragende Eigenschaft der erfindungsgemäß eingesetzten Copolymerwachse sind deren Verdickungsvermögen von Ölen.

Die in den erfindungsgemäßen Zubereitungen eingesetzten Copolymerwachse können durch radikalische Polymerisation hergestellt werden.

Die nachfolgenden Beispiele und Anwendungen sollen die Erfindung näher erläutern, ohne sie jedoch darauf zu beschränken (bei allen Prozentangaben handelt es sich um Gewichtsprozent).

### Herstellung von Copolymerwachsen

### Beispiel 1: Copolymerwachs 1

Copolymerwachs aus C₃₀₊-α-Olefinen, Acrylsäure und Acrylsäuremethylester

### Ansatz:

| | |
|---|---|
| C₃₀₊-α-Olefin | 2,20 mol |
| Acrylsäure | 0,33 mol |
| Acrylsäuremethylester | 5,17 mol |
| Di-tert.-Butylperoxid | 5 Gew.-% auf Acrylsäuremethylester |

### Herstellung:

Das Olefin wird bei 100°C aufgeschmolzen, der Ansatz auf 150°C erhitzt, dann Di-tert.-Butylperoxid, Acrylsäuremethylester und Acrylsäure unter Rückfluss zudosiert und 3 Stunden nachgerührt. Die freien Monomere werden abdestilliert.

| | |
|---|---|
| Säurezahl [mg KOH/g]: | 12,5 |

### Beispiel 2: Copolymerwachs 2

Copolymerwachs aus C_{26/28}-α-Olefinen, Acrylsäure und Acrylsäuremethylester

### Ansatz:

| | |
|---|---|
| C_{26/28}-α-Olefin | 2,00 mol |
| Acrylsäure | 0,20 mol |
| Acrylsäuremethylester | 3,80 mol |
| Di-tert.-Butylperoxid | 5 Gew.-% auf Acrylsäuremethylester |

### Herstellung:

Das Olefin wird bei 100°C aufgeschmolzen, der Ansatz auf 150°C erhitzt, dann Di-tert.-Butylester, Acrylsäuremethylester und Acrylsäure unter Rückfluss zudosiert und 3 Stunden nachgerührt. Die freien Monomere werden abdestilliert.

| | |
|---|---|
| Säurezahl [mg KOH/g]: | 10,2 |

### Beispiel 3: Copolymerwachs 3

Copolymerwachs aus C_{26/28}-α-Olefinen, Acrylsäure und Acrylsäuremethylester

### Ansatz:

| | |
|---|---|
| C_{26/28}-α-Olefin | 3,08 mol |
| Acrylsäure | 0,15 mol |
| Acrylsäuremethylester | 6,00 mol |
| Di-tert.-Butylperoxid | 5 Gew.-% auf Acrylsäuremethylester |

### Herstellung:

Das Olefin wird bei 100°C aufgeschmolzen, der Ansatz auf 150°C erhitzt, dann Di-tert.-Butylester, Acrylsäuremethylester und Acrylsäure unter Rückfluss zudosiert und 3 Stunden nachgerührt. Die freien Monomere werden abdestilliert.

| | |
|---|---|
| Säurezahl [mg KOH/g]: | 4,9 |

### Beispiel 4: Copolymerwachs 4

Copolymerwachs aus C₃₀₊-α-Olefinen, Acrylsäure und Acrylsäuremethylester

### Ansatz:

| | |
|---|---|
| C₃₀₊-α-Olefin | 1 mol |
| Acrylsäure | 0,3 mol |
| Acrylsäuremethylester | 2,5 mol |
| Di-tert.-Butylperoxid | 5 Gew.-% auf Acrylsäuremethylester |

### Herstellung:

Das Olefin wird bei 100°C aufgeschmolzen, der Ansatz auf 150°C erhitzt, dann Di-tert.-Butylester, Acrylsäuremethylester und Acrylsäure zudosiert und 5 Stunden nachgerührt. Die freien Monomere werden abdestilliert.

| | |
|---|---|
| Säurezahl [mg KOH/g]: | 5 |

### Beispiel 5: Copolymerwachs 5

Copolymerwachs aus C₃₀₊-α-Olefin und Acrylsäuremethylester

### Ansatz:

| | |
|---|---|
| C₃₀₊-α-Olefin | 1 mol |
| Acrylsäuremethylester | 1,5 mol |
| Di-tert.-Butylperoxid | 5 Gew.-% auf Acrylsäuremethylester |

### Herstellung:

Das Olefin wird bei 100°C aufgeschmolzen, der Ansatz auf 150°C erhitzt, dann Di-tert.-Butylperoxid und Acrylsäuremethylester zudosiert und 3 Stunden nachgerührt. Die freien Monomere werden abdestilliert.

| | |
|---|---|
| Säurezahl [mg KOH/g]: | 1 |

### Beispiel 6: Copolymerwachs 6

Mit Talgfettalkoholseitenketten modifiziertes Copolymerwachs

| | |
|---|---|
| Copolymerwachs 5 | 1 mol |
| Talgfettalkohol | 0,5 mol |
| Na-Methylat | 0,3 Gew.-% auf Ansatz |

### Herstellung:

Das Copolymerwachs 5 wird bei 100°C aufgeschmolzen, mit dem Katalysator (Na-Methylat) und der Alkoholkomponente (Talgfettalkohol) versetzt und auf 180°C erhitzt. Bei dieser Temperatur wird 7 Stunden gerührt und freiwerdendes Methanol abdestilliert. Dann wird Vakuum angelegt, um Reste an Methanol und andere Alkohole abzudestillieren. Es wird mit Phosphorsäure neutralisiert, auf 120°C abgekühlt und filtriert.

| | |
|---|---|
| Säurezahl [mg KOH/g]: | 10 |

### Beispiel 7: Gopolymerwachs 7

Mit Perfluoralkylpropanol-(C₁₂-C₁₄)-seitenketten modifiziertes Copolymerwachs

| | |
|---|---|
| Copolymerwachs 4 | 1 mol |
| C₁₂-C₁₄-Perfluoralkylpropanol | 0,12 mol |
| Na-Methylat | 0,3 Gew.-% auf Ansatz |

### Herstellung:

Das Copolymerwachs 4 wird bei 100°C aufgeschmolzen, mit dem Katalysator (Na-Methylat) und der Alkoholkomponente C₁₂-C₁₄-Perfluoralkylpropanol [CF₃(CF₂)₁₀₋₁₂-CF₂-CH₂-CH₂-CH₂-OH] versetzt und auf 180°C erhitzt. Bei dieser Temperatur wird 7 Stunden gerührt und freiwerdendes Methanol abdestilliert. Dann wird Vakuum angelegt um Reste an Methanol und andere Alkohole abzudestillieren. Es wird mit Phosphorsäure neutralisiert, auf 120°C abgekühlt und filtriert.

| | |
|---|---|
| Säurezahl [mg KOH/g]: | 10 |

### Beispiel 8: Copolymerwachs 8

Mit Triacetondiaminseitenketten modifiziertes Copolymerwachs

| | |
|---|---|
| Copolymerwachs 4 | 1 mol |
| Triacetondiamin | 0,25 mol |
| Fascat® 4102 | 0,2 Gew.-% auf Ansatz |
| (Butyl-Zinn-Tris-2-Ethylhexoat) | |

### Herstellung:

Das Copolymerwachs 4 wird bei 100°C aufgeschmolzen, mit dem Katalysator (Fascat® 4102) und der Aminkomponente versetzt und auf 190°C erhitzt. Bei dieser Temperatur wird 7 Stunden gerührt und freiwerdendes Methanol abdestilliert. Dann wird Vakuum angelegt um Reste an Methanol und Amin abzudestillieren. Es wird auf 120°C abgekühlt und filtriert.

| | |
|---|---|
| Säurezahl [mg KOH/g]: | 10 |

### Beispiel 9: Copolymerwachs 9

Mit 2,2,6,6-Tetramethylpiperidinol seitenkettenmodifiziertes Copolymerwachs

| | |
|---|---|
| Copolymerwachs 4 | 1 mol |
| 2,2,6,6 Tetramethylpiperidinol | 0,6 mol |
| Fascat® 4102 (Butyl-Zinn-Tris-2-Ethylhexoat) | 0,2 Gew.-% auf Ansatz |

### Herstellung:

Das Copolymerwachs 4 wird bei 100°C aufgeschmolzen, mit dem Katalysator und der Aminkomponente versetzt und auf 190°C erhitzt. Bei dieser Temperatur wird 7 Stunden gerührt und freiwerdendes Methanol abdestilliert. Dann wird Vakuum angelegt um Reste an Methanol und Amin abzudestillieren. Es wird auf 150°C abgekühlt und filtriert.

| | |
|---|---|
| Säurezahl [mg KOH/g]: | 8 |

### Beispiel 10: Copolymerwachs 10

Mit Octylamin seitenkettenmodifiziertes Copolymerwachs

| | |
|---|---|
| Copolymerwachs 4 | 1 mol |
| Octylamin | 0,6 mol |
| Fascat® 4102 (Butyl-Zinn-Tris-2-Ethylhexoat) | 0,2 Gew.-% auf Ansatz |

### Herstellung:

Das Copolymerwachs 4 wird bei 100°C aufgeschmolzen, mit dem Katalysator und der Aminkomponente versetzt und auf 190°C erhitzt. Bei dieser Temperatur wird 7 Stunden gerührt und freiwerdendes Methanol abdestilliert. Dann wird Vakuum angelegt um Reste an Methanol und Amin abzudestillieren. Es wird auf 150°C abgekühlt und filtriert.

| | |
|---|---|
| Säurezahl [mg KOH/g]: | 16 |

### Anwendungsbeispiel

### Beispiel A: Lippenstiftformulierung

Copolymerwachse 1, 2 und 3 sowie Beeswax wurden in Basisformulierungen für Lippenstifte eingearbeitet. Die Mengen der Inhaltsstoffe sind in Gew.-% angegeben. Es wurden der Erweichungspunkt, die Glastemperatur, die Nadelpenetrationszahl sowie die Härte der resultierenden Zusammensetzung bestimmt.

Die Ergebnisse sind in Tabelle 1 dargestellt.

**Tabelle 1: Lippenstiftformulierung**

| | | | | |
|---|---|---|---|---|
| Crodamol PMP | 17,42 | 17,42 | 17,42 | 17,42 |
| Novol | 34,27 | 34,27 | 34,27 | 34,27 |
| Fluilan | 5,62 | 5,62 | 5,62 | 5,62 |
| Crodesta F-50 | 5,06 | 5,06 | 5,06 | 5,06 |
| Castor oil | 19,55 | 19,55 | 19,55 | 19,55 |
| Butylhydroxyanisol | 0,11 | 0,11 | 0,11 | 0,11 |
| Copolymerwachs 1 | 17,98 | - | - | - |
| Copolymerwachs 2 | - | 17,98 | - | - |
| Copolymerwachs 3 | - | - | 17,98 | - |
| Bees wax | - | - | - | 17,98 |
| Total | 100 | 100 | 100 | 100 |
| EP [°C] | 49 | 50 | 48 | 52 |
| Pastenhärte [g/cm²] | 2090 | 670 | 600 | 2975 |

### EP: Erweichungspunkt

Die Ergebnisse der Tabelle 1 zeigen, dass insbesondere Copolymerwachs 1 aufgrund der resultierenden Härte der Formulierung zur Herstellung von Lippenstiften sehr gut geeignet ist.

Es wurden folgende Meßmethoden verwendet:

| | |
|---|---|
| Säurezahl: | ISO 2114 |
| Schmelzpunkt: | Differential Scanning Calorimetry ISO 11357-1/2/3 |
| Erweichungspunkt: | DIN 51920, ASTM D 3104 |

### Pastenhärte:

Die Pastenhärte gibt die Masse in Gramm an, mit der ein Stempel mit der Fläche von 1 cm² belastet werden muß, um in die Paste einzudringen. Beschrieben ist diese Methode in Seifen-Öle-Fette-Wachse, 83, S. 595 (1957).

### Vergleichbeispiel V1: Copolymerwachs V1

Copolymerwachs aus C₁₈-α-Olefinen, Acrylsäure und Acrylsäuremethylester

### Ansatz:

| | |
|---|---|
| C₁₈-α-Olefin | 3,15 mol |
| Acrylsäure | 0,32 mol |
| Acrylsäuremethylester | 5,99 mol |
| Di-tert.-Butylperoxid | 5 Gew.-% auf Acrylsäuremethylester |

### Herstellung:

Das Olefin wird bei 100°C aufgeschmolzen, der Ansatz auf 150°C erhitzt, dann Ditert.-Butylester, Acrylsäuremethylester und Acrylsäure unter Rückfluss im Laufe vo 3 Stunden zudosiert und 3 Stunden nachgerührt. Die freien Monomere werden abdestilliert.

| | |
|---|---|
| Säurezahl [mg KOH/g]: | 12,0 |

**Tabelle 2: Viskositäten von Copolymerwachsen aus C₃₀₊-α-Olefin, C_{26/28}-α-Olefin und C₁₈-α-Olefin in Olivenöl/Rizinusöl (10 Gew.-% Copolymerwachs; 90 Gew.-% Olivenöl/Rizinusöl)**

| Copolymerwachs | α-Olefin | Viskosität [mPas] | dV/Upm |
|---|---|---|---|
| Copolymerwachs 1 | C₃₀₊-α-Olefin | 930 | 5,08 |
| Copolymerwachs 2 | C_{26/28}-α-Olefin | 800 | 4,12 |
| Copolymerwachs V1 | C₁₈-α-Olefin | 320 | 0,44 |

Die Viskositätsmessungen erfolgten bei 25°C im Platten-Kegel Viskosimeter. Nach der Methode DIN 53018 wurden die Viskositäten bei Drehzahlen (rotierender Kegel auf feststehender Platte) von 12,80 bis 35,80 (1/min) gemessen. Die in der Tabelle 2 angegebenen Viskositätswerte in mPas sind Durchschnittswerte, die bei Drehzahlen von 23,33 (1 /min) erhalten wurden.

Die Werte dV/Upm stellen die Differenzen der gemessenen Viskositäten bei der Drehzahl 12,80 (1/min) und 35,80 (1/min) dar.

Copolymerwachse aus kürzerkettigen α-Olefinen (C₁₈-α-Olefin) in Olivenöl/Rizinusöl haben geringere Viskositäten, die Viskosität nimmt mit steigender Scherung nur wenig ab.

Copolymerwachse aus längerkettigen α-Olefinen (C_{26/28}-α-Olefin, C₃₀₊-α-Olefin) in Olivenöl/Rizinusöl haben höhere Viskositäten und zeigen ein ausgeprägtes strukturviskoses Verhalten, d. h. die Viskositäten nehmen mit steigender Scherkraft, hervorgerufen durch steigende Drehzahlen des Messkörpers, ab. Die Produkte sind im Ruhestand - ohne Scherung - gelartig.

### Formulierungsbeispiele

### Beispiel A: W/O-Creme

| | | | |
|---|---|---|---|
| A | Hostacerin® DGI | | 4,00 % |
| | Copolymerwachs 3 | Clariant | 5,00 % |
| | Magnesiumstearat | | 1,00 % |
| | Mineralöl, niedrigviskos | | 5,00 % |
| | Vaseline | | 10,00 % |
| | Cetiol® V | | 5,00 % |
| | | | |
| B | 1,2-Propylenglycol | | 3,00 % |
| | Wasser dest. | | ad 100 % |
| | Konservierungsmittel | | q.s. |
| | | | |
| C | Duftstoff | | 0,40 % |

### Herstellung:

- I: Schmelzen von A bei 80°C
- II: Erwärmen von B auf 80°C
- III: Einrühren von II in I
- IV: Rühren bis Temperatur von 35°C erreicht ist
- V: Zugabe von C zu IV bei 35°C

### Beispiel B: O/W-Creme

| | | | |
|---|---|---|---|
| A | Hostacerin® DGI | | 2,00 % |
| | SilCare® Silicone 31 M40 | Clariant | 4,00 % |
| | Perliquidum | | 4,00 % |
| | Copolymerwachs 6 | Clariant | 2,00 % |
| | Eutanol G | Clariant | 4,00 % |
| | Isopropylpalmitat | Clariant | 4,00 % |
| | Carbopol 980 | | 0,70 % |
| | | | |
| B | Hostapon® KCG | | 0,60 % |
| | Natronlauge (10 % in Wasser) | | 2,10 % |
| | Konservierungsmittel | | q.s. |
| | Duftstoff | | 0,40 % |
| | Wasser dest. | | ad 100 % |

### Herstellung:

- I: Erwärmen von A auf 80°C
- II: Erwärmen von B auf 80°C
- III: Emulgieren durch langsames Einrühren von B in A.

### Beispiel C: Haarkur

| | | |
|---|---|---|
| A | Wasser dest. | ad 100 % |
| | | |
| B | Genamin® KSL | 7 % |
| | Hostaphat® KL 340 D | 1,5 % |
| | Genapol® PDB | 4 % |
| | Copolymerwachs 1 | 1 % |
| | Jojobaöl | 1 % |
| | Propylenglykol | 0,8 % |
| | Isopropylpalmitat | 1 % |
| | Dow Corning® 190 | 0,8 % |
| | Extrapon | 0,3 % |
| | Vitamin E | 0,3 % |
| | Panthenol (Vitamin B 5) | 0,5 % |
| | | |
| C | Zitronensäure (50 %ig in Wasser) | 0,2 % |

### Herstellung:

- I: A auf 75°C erwärmen
- II: B auf 75°C erwärmen
- III: A unter Rühren zu B geben und Kaltrühren
- IV: mit C auf pH 6 einstellen

### Beispiel D: Cremespülung

| | | |
|---|---|---|
| A | Tylose® H 100 000 YP2 | , 1,5 % |
| | Wasser dest. | ad 100 % |
| | | |
| B | Genamin® KSL | 7 % |
| | Hostaphat® KL 340 D | 1,5 % |
| | Genapol® PDB | 4 % |
| | Copolymerwachs 2 | 1 % |
| | | |
| C | Zitronensäure | 0,2 % |

### Herstellung:

- I: Tylose im Wasser bei Raumtemperatur unter Rühren aufquellen
- II: I auf 75°C erwärmen
- III: B auf 75°C erwärmen
- IV: II zu III geben und kaltrühren
- V: mit C auf pH 6 einstellen.

### Beispiel E: Antiperspirant

| | | | |
|---|---|---|---|
| A | Locron® L | Clariant | 10,00 % |
| | Ethanol | | 50,00 % |
| | Farnesol | | 0,50 % |
| | Duftstoff | | 0,20 % |
| | Copolymerwachs 6 | | 0,50 % |
| | Wasser dest. | | ad 100 % |
| | Extrapon Avocado special | | 1,50 % |

### Herstellung:

Mischen der Komponenten A

### Beispiel F: W/O-Antiperspirant Creme

| | | |
|---|---|---|
| A | Abil EM90 | 2,0 % |
| | Abil B8839 | 20,0 % |
| | Copolymerwachs 3 | 2,0 % |
| | | |
| B | Aloxicoll L | 17,0 % |
| | Wasser dest. | ad 100 % |
| | Parfümöl | q.s. |
| | Konservierungsmittel | q.s. |

### Herstellung:

- I: Phase B langsam unter Rühren bei Raumtemperatur zur Phase A geben
- II: Homogenisieren

### Beispiel G: Klarer Deo-Stick

| | | | |
|---|---|---|---|
| A | OCTOPIROX® | (Clariant) | 0,10 % |
| | Copolymerwachs 3 | | 2,00 % |
| | Propylene Glycol | | 71,00 % |
| | Rewoderm 66E | | 5,00 % |
| | Natriumstearat | | 5,00 % |
| | Genapol® HS 020 | (Clariant) | 1,00 % |
| | Wasser dest. | | ad 100 % |

### Herstellung:

- I: Mischen der Komponenten A und bei ca. 50°C rühren bis die Lösung klar ist
- II: Abfüllen und Abkühlen

### Beispiel H: Alkoholfreier Deodorant-Roll-on (opak)

| | | |
|---|---|---|
| A | Tegodeo CW 90 | 2,0 % |
| | Polyethylenglycol(3)laurylether | 1,0 % |
| | Triethanolamin | 1,0 % |
| | | |
| B | Copolymerwachs 1 | 1,2 % |
| | Wasser dest. | ad 100 % |
| | | |
| C | Tagat R 40 | 3,0 % |
| | Parfümöl | q.s. |
| | Konservierungsmittel | q.s. |
| | | |
| D | Zitronensäure | 0,2 % |

### Herstellung:

- I: Phasen A und B separat auf 80°C erwärmen
- II: Phase B in Phase A einrühren und homogenisieren
- III: Unter langsamem Rühren abkühlen
- IV: Bei 30°C Phase C hinzufügen
- V: Den pH-Wert mit Hilfe von Phase D einstellen

### Beispiel I: Sonnenschutz-Gel

| | | | |
|---|---|---|---|
| A | Crodamol® AB | | ad 100 % |
| | Neo Heliopan® AV | | 7,50 % |
| | SilCare® Silicone 41 M80 | (Clariant) | 5,00 % |
| | Copolymerwachs 6 | | 1 % |
| | | | |
| B | Neo Heliopan® BB | | 3,00 % |

### Herstellung:

- I: A auf ca. 80°C erwärmen
- II: B in A lösen
- III: Abfüllen und auf 25°C abkühlen ohne rühren

### Beispiel J: Grundierung

| | | | |
|---|---|---|---|
| A | Nexbase® 2004 FG | | 9,00 % |
| | Myritol® 318 | | 5,00 % |
| | Almond Oil | | 4,00 % |
| | SilCare® Silicone 31 M40 | (Clariant) | 4,00 % |
| | SilCare® Silicone 41 M15 | (Clariant) | 3,00 % |
| | Genapol® HS020 | (Clariant) | 1,60 % |
| | Genapol® HS200 | (Clariant) | 2,40 % |
| | Copolymerwachs 3 | | 1,00 % |
| | | | |
| B | Vanclay® | | 1,50 % |
| | Talc | | 3,00 % |
| | Iron Oxide Pigments | | 7,90 % |
| | | | |
| C | Glycerin | | 5,00 % |
| | Wasser dest. | | ad 100 % |
| | Aristoflex® AVC | (Clariant) | 0,40% |
| D | Duftstoff | | q.s. |
| | Nipaguard® PDU | (Clariant) | q.s. |

### Herstellung:

- I: Mischen und schmelzen der Komponenten A bei ca. 70°C
- II: Zugabe von B zu I bei ca. 70°C unter Rühren
- III: Mischen von C bis Aristoflex® AVC gelöst ist und Erwärmen auf ca. 70°C
- IV: Zugabe von C zu II unter Rühren und Homogenisieren
- V: Zugabe von D zu IV bei < 40°C

### Beispiel K: Mascara

| | | | |
|---|---|---|---|
| A | Tylose® H 4000 G4 | (Clariant) | 0,70 % |
| | 1,2-Propylenglykol | | 1,00% |
| | Wasser dest. | | ad 100 % |
| | | | |
| B | Triethanolamine 99% | | 1,20 % |
| | | | |
| C | Stearinsäure | | 3,00 % |
| | SilCare® Silicone 41 M15 | (Clariant) | 1,00 % |
| | SilCare® Silicone 31 M40 | (Clariant) | 2,00 % |
| | Tegocare® 450 | | 4,00 % |
| | Nexbase® 2006 | | 2,00 % |
| | Bienenwachse | | 2,50 % |
| | Candelilla Wachs | | 2,50 % |
| | Copolymerwachs 2 | | 3,50 % |
| | | | |
| D | Pigmente | | 10,00 % |
| | | | |
| E | Nipagin® M | (Clariant) | 0,20 % |
| | Nipasol® M | (Clariant) | 0,10 % |
| F | Duftstoff | | q.s. |

### Herstellung:

- I: Komponenten A bei Raumtemperatur unter Rühren aufquellen; auf 85°C erwärmen.
- II: Zugabe von B zu A und rühren
- III: Schmelzen der Komponenten D auf ca. 85°C
- IV: Zugabe von D zu III unter Rühren bei 85°C
- V: Zugabe von II zu IV unter kräftigem Rühren (ca. 15 Minuten bei 85°C, weitere 15 Minuten ohne Erwärmen)
- VI: Zugabe von E und F zu V bei ca. 35 bis 40°C
- VII: Abfüllen bei 35 bis 40°C.

### Beispiel L: Tensidfreie Lotion mit erfrischender, belebender Wirkung

| | | |
|---|---|---|
| A | Jojoba oil | 2,00 % |
| | Almondöl | 3,00 % |
| | Cetiol® V | 3,00 % |
| | | |
| B | Copolymerwachs 2 | 2,00 % |
| | | |
| C | Glycerin | 3,00 % |
| | Menthol | 0,70 % |
| | Campher | 0,30 % |
| | Ethanol | 5,00 % |
| | Wasser dest. | ad 100 % |
| | Konservierungsmittel | q.s. |
| | | |
| D | Duftstoff | 0,30 % |
| | | |
| E | Zitronensäure | q.s. |

### Herstellung:

- I: A und B mischen
- II: Lösung von C in I einrühren
- III: D zu II geben
- IV: Homogenisieren
- V: pH mit Hilfe von Phase E auf 6,00 einstellen.

### INCI Bezeichnung der eingesetzten Handelsprodukte:

| | | |
|---|---|---|
| Abil B8839 | | Cyclopentasiloxan/Cyclohexasiloxan |
| Abil EM90 | | Cetyldimethicon/Copolyol |
| Aloxicoll L. | | Alumiunium Chlorohydrat |
| Aristoflex® AVC | (Clariant) | Ammonium Acyloyldimethyltaurate/VP Copolymer |
| Carbopol 980 | | Polyacrylat |
| Cetiol® V | (Cognis) | Decyloleat |
| Crodamol® AB | | C₁₂₋₁₅ Alkyl Benzoate |
| Dow Corning® 190 | (Dow Corning) | Dimethicon Copolyol |
| Euperlan® PK 3000 | (Henkel) | Glycol Distearat, Laureth-4, Cocamidopropyl Betain |
| Eutanol G | | 2-Octyldodecanol |
| Extrapon® | (Dragoco) | Pflanzliche Extrakte |
| Extrapon Avocado special | | Wasser/ Ethoxydiglycol/ Propylenglycol/Butylenglycol/Persea Gratissima Extrakt |
| Genamin® CTAC | (Clariant) | Cetyltrimethylammonium Chlorid |
| Genamin® KDM-P | (Clariant) | Behenyltrimethylammonium Chlorid |
| Genamin® KSL | (Clariant) | PEG- 5 Stearyl Ammonium Lactat |
| Genamin® STAC | (Clariant) | Stearytrimethylammonium Chlorid |
| GENAPOL® HS 020 | (Clariant) | Steareth-2 |
| Genapol® HS200 | (Clariant) | Steareth-20 |
| Genapol® PDB | (Clariant) | Glycol Distearat, Laureth- 4, |
| | | Cocamidopropyl Betain |
| Hostacerin® DGI | | Polyglyceryl-2-Sesquiisostearat |
| Hostacerin® T- 3 | | Ceteareth-3 |
| Hostaphat® KL 340 D | | Trilaureth-4 Phosphat |
| Hostapon® KCG | | Natriumcocoylglutamat |
| Locron® L | (Clariant) | Aluminium Chlorohydrat |
| Myritol® 318 | | Capric/Caprylic Triglyceride |
| Neo Heliopan® AV | | Ethylhexyl Methoxycinnamate |
| Neo Heliopan® BB | | Benzophenone-3 |
| Nexbase® 2004 FG | | Hydrogenated Poly-1-Decene |
| Nexbase® 2006 | | Poly-1-Decen |
| Nipagin® M | (Clariant) | Methylparaben |
| NIPAGUARD® CMB | (Clariant) | Triethylenglycol/ Benzylalkohol/Propylenglycol/ |
| Nipaguard® PDU | (Clariant) | Propylene Glycol (and) Diazolidinyl Urea (and) Methylparaben (and) Propylparaben |
| Nipasol® M | (Clariant) | Propylparaben |
| OCTOPIROX® | (Clariant) | Piroctone Olamine |
| Perliquidum | | Paraffinöl |
| Rewoderm 66E | | Isostearate |
| SilCare® Silicone 31 M40 | (Clariant) | Caprylyl Trimethicone |
| SilCare® Silicone 31 M50 | (Clariant) | Caprylyltrimethicon |
| SilCare® Silicone 41 M15 | (Clariant) | Caprylylmethicon |
| SilCare® Silicone 41 M80 | (Clariant) | C₂₄₋₂₈ Alkyl Dimethicone |
| Tagat R40 | | PEG-40 hydrogeniertes Castoröl |
| Tegocare® 450 | | Polyglyceryl-3 Methylglucose Distearat |
| Tegodeo CW 90 | | Zinkricinoleat/ Tetrahydroxypropyl-Ethylendiamin/Laureth-3/ Propylenglycol |
| Tylose® H 100000 YP2 | | Hydroxyethylcellulose |
| Tylose® H 4000 G4 | | Hydroxyethylcellulose |
| Vanclay® | | Kaolin |

## Patentansprüche

1. Kosmetische, pharmazeutische oder dermatologische Zubereitung, **dadurch gekennzeichnet, dass** sie ein oder mehrere Copolymerwachse, enthaltend
a) eine oder mehrere Struktureinheiten -CH₂-CHR-, wobei R für eine lineare oder verzweigte Alkylgruppe mit 24 bis 58 Kohlenstoffatomen steht,
b) gegebenenfalls eine oder mehrere Struktureinheiten 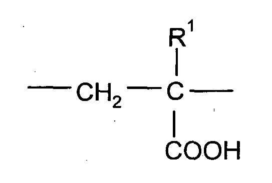 wobei R¹ für Wasserstoff oder Methyl steht,
c) eine oder mehrere Struktureinheiten 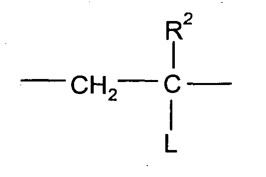 wobei
R² für Wasserstoff oder Methyl steht,
L -COOR³, -CONR⁷R⁸ oder -COO⁻X⁺ bedeutet,
R³ für eine geradkettigte oder verzweigte Alkylgruppe mit 1 bis 36 Kohlenstoffatomen oder eine geradkettigte oder verzweigte Alkenylgruppe mit 2 bis 36 Kohlenstoffatomen steht, die gegebenenfalls auch alkoxyliert sein kann und vorzugsweise Ethylenoxy- (EO), Propylenoxy- (PO), Butylenoxy- (BO) oder EO/PO-Gruppen enthalten kann, oder
eine Gruppe (AO)ₓ-H ist, wobei (AO) für eine Ethoxy-, Propoxy- oder Butoxygruppe steht und x eine Zahl von 1 bis 50 ist, oder
für eine Glycidylgruppe, eine C₂-C₁₀-Hydroxyalkylgruppe oder für eine Glyceringruppe steht, oder
für eine cyclische aromatische oder nichtaromatische Gruppe, vorzugsweise eine Cycloalkylgruppe, mit 5 bis 8, vorzugsweise 6, Ringatomen steht, oder
für eine cyclische aromatische oder nichtaromatische Gruppe mit 5 bis 8, vorzugsweise 6, Ringatomen steht, wobei der Ring aus Kohlenstoffatomen und Heteroatomen, vorzugsweise O und/oder N, gebildet ist, und sowohl die Kohlenstoffatome, als auch die Stickstoffatome mit linearen oder verzweigten Alkyl- oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁴, worin R⁴ eine Alkylgruppe mit 1 bis 22 Kohlenstoffatomen ist, substituiert sein können, oder
für -(CR⁵R⁶)y-Cycloalkyl oder für -(CR⁵R⁶)_{y}-Aryl steht, wobei R⁵ und R⁶ jeweils unabhängig voneinander für H oder eine lineare oder verzweigte Alkylgruppe mit 1 bis 4 Kohlenstoffatomen stehen und y eine Zahl von 1 bis 10 ist, oder
für eine Perfluoralkylgruppe mit 8 bis 18 Kohlenstoffatomen steht,
R⁷ und R⁸ jeweils unabhängig voneinander
für Wasserstoff stehen, oder
für eine geradkettigte oder verzweigte Alkylgruppe mit 1 bis 36 Kohlenstoffatomen oder eine geradkettigte oder verzweigte Alkenylgruppe mit 2 bis 36 Kohlenstoffatomen stehen, die gegebenenfalls auch alkoxyliert sein können und vorzugsweise Ethylenoxy- (EO), Propylenoxy- (PO), Butylenoxy- (BO) oder EO/PO- Gruppen enthalten können, oder
für eine (C₂-C₁₀)-Hydroxyalkylgruppe stehen, oder
für -CH₂-CH₂-N(CH₃)₂ oder für einen Polyaminrest stehen, oder
für eine cyclische aromatische oder nichtaromatische Gruppe, vorzugsweise eine Cycloalkylgruppe, mit 5 bis 8, vorzugsweise 6, Ringatomen stehen, oder
für eine cyclische aromatische oder nichtaromatische Gruppe mit 5 bis 8, vorzugsweise 6, Ringatomen stehen, wobei die Ringe aus Kohlenstoffatomen und Heteroatomen, vorzugsweise O und/oder N, gebildet sind, und sowohl die Kohlenstoffatome, als auch die Stickstoffatome mit linearen oder verzweigten Alkyl- oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁹, worin R⁹ eine Alkylgruppe mit 1 bis 22 Kohlenstoffatomen ist, substituiert sein können, oder
R⁷ und R⁸ zusammen mit dem Stickstoffatom, an das sie gebunden sind, einen 5-, 6- oder 7-gliedrigen aromatischen oder nichtaromatischen Ring bilden, und die Ringe neben dem Stickstoffatom vorzugsweise lediglich CH₂-Gruppen enthalten,
X⁺ für Li⁺, Na⁺, K⁺, Mg⁺⁺/2, Ca⁺⁺/2, Al⁺⁺⁺/3, NH₄⁺, ein Monoalkylammonium-, Dialkylammonium-, Trialkylammonium- und/oder Tetraalkylammoniumion steht, wobei es sich bei den Alkylsubstituenten der Ammoniumionen unabhängig voneinander um (C₁-C₂₂)-Alkylreste oder (C₂-C₁₀)-Hydroxyalkylreste handeln kann, und
d) gegebenenfalls eine oder mehrere Struktureinheiten, die sich von Styrol, 3-Methylstyrol, 4-Methylstyrol oder α-Methylstyrol ableiten,
enthält.

2. Zubereitung nach Anspruch 1, **dadurch gekennzeichnet, dass** die Copolymerwachse Struktureinheiten der Komponente a) enthalten, worin R für eine lineare oder verzweigte Alkylgruppe mit 24 und/oder 26 Kohlenstoffatomen steht.

3. Zubereitung nach Anspruch 1, **dadurch gekennzeichnet, dass** die Copolymerwachse Struktureinheiten der Komponente a) enthalten, worin R für eine lineare oder verzweigte Alkylgruppe mit 28 bis 58 Kohlenstoffatomen steht.

4. Zubereitung nach einem oder mehreren der Ansprüche 1 bis 3, **dadurch gekennzeichnet, dass** die Copolymerwachse Struktureinheiten der Komponente c) enthalten, worin R² Wasserstoff oder Methyl, vorzugsweise Wasserstoff, bedeutet, L -COOR³ ist und R³ für eine cyclische aromatische oder nichtaromatische Gruppe mit 5 bis 8, vorzugsweise 6, Ringatomen steht, wobei der Ring aus Kohlenstoffatomen und Heteroatomen, vorzugsweise O und/oder N, gebildet ist, und sowohl die Kohlenstoffatome, als auch die Stickstoffatome mit linearen oder verzweigten Alkyl- oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁴, worin R⁴ eine Alkylgruppe mit 1 bis 22 Kohlenstoffatomen ist, substituiert sein können.

5. Zubereitung nach Anspruch 4, **dadurch gekennzeichnet, dass** R³ ein Rest ist, der von Tetramethylpiperidinol, 2,2,6,6-Tetramethylpiperidinol, N-Methyl-2,2,6,6-tetramethylpiperidinol, N-Acetyl-2,2,6,6-tetramethylpiperidinol und/oder 2,2,6,6-Tetramethylpiperidinol-N-Oxid abgeleitet ist.

6. Zubereitung nach einem oder mehreren der Ansprüche 1 bis 3, **dadurch gekennzeichnet, dass** die Copolymerwachse Struktureinheiten der Komponente c) enthalten, worin R² Wasserstoff oder Methyl, vorzugsweise Wasserstoff, bedeutet, L -COOR³ ist und R³ für eine geradkettigte oder verzweigte Alkylgruppe mit 1 bis 36 Kohlenstoffatomen oder eine geradkettigte oder verzweigte Alkenylgruppe mit 2 bis 36 Kohlenstoffatomen steht, die gegebenenfalls auch alkoxyliert sein kann, vorzugsweise mit 1 bis 30 Alkoxyeinheiten und insbesondere Ethylenoxy- (EO), Propylenoxy- (PO), Butylenoxy- (BO) oder EO/PO-Gruppen enthalten kann.

7. Zubereitung nach Anspruch 6, **dadurch gekennzeichnet, dass** R³ für eine geradkettigte oder verzweigte Alkylgruppe mit 1 bis 13, vorzugsweise mit 1 bis 10 und besonders bevorzugt mit 1 bis 8 Kohlenstoffatomen steht.

8. Zubereitung nach Anspruch 6, **dadurch gekennzeichnet, dass** R³ für eine geradkettigte oder verzweigte Alkylgruppe mit 14 bis 36, vorzugsweise mit 14 bis 30 und besonders bevorzugt mit 14 bis 22 Kohlenstoffatomen steht.

9. Zubereitung nach einem oder mehreren der Ansprüche 1 bis 3, **dadurch gekennzeichnet, dass** die Copolymerwachse Struktureinheiten der Komponente c) enthalten, worin R² Wasserstoff oder Methyl, vorzugsweise Wasserstoff, bedeutet, L -COOR³ ist und R³ für eine Perfluoralkylgruppe mit 8 bis 18 Kohlenstoffatomen steht.

10. Zubereitung nach einem oder mehreren der Ansprüche 1 bis 3, **dadurch gekennzeichnet, dass** die Copolymerwachse Struktureinheiten der Komponente c) enthalten, worin R² Wasserstoff oder Methyl, vorzugsweise Wasserstoff, bedeutet, L -CONR⁷R⁸ ist und R⁷ und R⁸ die in Anspruch 1 angegebene Bedeutung besitzen.

11. Zubereitung nach einem oder mehreren der Ansprüche 1 bis 10, **dadurch gekennzeichnet, dass** die Copolymerwachse die Komponenten a), b) und c) in den molaren Verhältnissen Komponente a): Komponente b) : Komponente c) von 1 : 0 bis 2 : 1 bis 4, vorzugsweise 1 : 0 bis 0,5 : 1,5 bis 3 und besonders bevorzugt 1 : 0,1 bis 0,25 : 2,0 bis 2,5 enthalten.

12. Zubereitung nach Anspruch 11, **dadurch gekennzeichnet, dass** R in der Struktureinheit von Komponente a) für eine lineare oder verzweigte Alkylgruppe mit 24 und/oder 26 Kohlenstoffatomen steht und die Copolymerwachse die Komponenten a), b) und c) in den molaren Verhältnissen Komponente a) : Komponente b) : Komponente c) von 1 : 0 bis 1 : 1 bis 3, vorzugsweise 1 : 0 bis 0,5 : 1,5 bis 3 und besonders bevorzugt 1 : 0,1 bis 0,25 : 2,0 bis 2,5 enthalten.

13. Zubereitung nach Anspruch 11, **dadurch gekennzeichnet, dass** R in der Struktureinheit von Komponente a) für eine lineare oder verzweigte Alkylgruppe mit 28 oder mehr Kohlenstoffatomen steht und die Copolymerwachse die Komponenten a), b) und c) in den molaren Verhältnissen Komponente a) : Komponente b) : Komponente c) von 1 : 0 bis 2 : 1 bis 4, vorzugsweise 1 : 0 bis 0,5 : 1,5 bis 3 und besonders bevorzugt 1 : 0,1 bis 0,25 : 2,25 bis 2,4 enthalten.

14. Zubereitung nach einem oder mehreren der Ansprüche 1 bis 13, **dadurch gekennzeichnet, dass** die Copolymerwachse Molekulargewichte im Bereich von 1 000 bis 500 000, vorzugsweise im Bereich von 1 500 bis 150 000 und besonders bevorzugt im Bereich von 1 500 bis 100 000 haben.

15. Zubereitung nach einem oder mehreren der Ansprüche 1 bis 14, **dadurch gekennzeichnet, dass** es sich um einen Abdeckstift, Akne-Stift, Lippenstift, ein Make-up, eine Grundierung, ein Gesichtspuder, Rouge, eine Mascara, einen Lidschatten, Eye-liner, eine Peeling-Creme, ein Haarwachs, Haar-Stylingmittel, Styling-Fluid, einen Haarschaum, ein Haargel, Haarspray, eine Mousse, ein Haaröl und Spitzenfluid, eine Haarkur, Nachtcreme, Pflegecreme, Nährcreme, Bodylotion, Salbe, ein Lippenpflegemittel, Sonnenschutzmittel, Deodorant, Antiperspirant, coloriertes Gel in Form eines Stifts wie z.B. eines Mehrphasenstifts, einen Stick, eine Paste, ein Puder, eine Creme, einen Cremeschaum, eine Lotion, eine selbstschäumende, schaumförmige, nachschäumende oder schäumbare Emulsion, ein Gel, Roll-On-Präparat oder einen Schaum handelt.

16. Zubereitung nach einem oder mehreren der Ansprüche 1 bis 15, **dadurch gekennzeichnet, dass** sie in Form einer Emulsion vorliegt.

17. Zubereitung nach Anspruch 16, **dadurch gekennzeichnet, dass** sie in Form einer Öl-in-Wasser-Emulsion vorliegt.

18. Zubereitung nach Anspruch 17, **dadurch gekennzeichnet, dass** sie als Emulsion vom Typ Öl-in-Wasser, vorzugsweise als kosmetische oder dermatologische Emulsion vom Typ Öl-in-Wasser, vorliegt und bezogen auf das Gesamtgewicht der Zubereitung
a) bis zu 95 Gew.-%, vorzugsweise 60 bis 92 Gew.-%, besonders bevorzugt 70 bis 90 Gew.-%, insbesondere bevorzugt 75 bis 85 Gew.-% einer Wasserphase,
b) bis zu 40 Gew.-%, vorzugsweise 1 bis 40 Gew.-%, besonders bevorzugt 2 bis 25 Gew.-%, insbesondere bevorzugt 5 bis 20 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs
enthält.

19. Zubereitung nach Anspruch 17, **dadurch gekennzeichnet, dass** sie als Gelcreme vom Typ Öl-in-Wasser, vorzugsweise als kosmetische oder dermatologische Gelcreme vom Typ Öl-in-Wasser, vorliegt und bezogen auf das Gesamtgewicht der Zubereitung
a) bis zu 95 Gew.-%, vorzugsweise 50 bis 95 Gew.-%, besonders bevorzugt 70 bis 90 Gew.-%, insbesondere bevorzugt 75 bis 85 Gew.-% einer Wasserphase,
b) bis zu 30 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) bis zu 5 Gew.-%, vorzugsweise 0,5 bis 5 Gew.-%, besonders bevorzugt 0,2 bis 4 Gew.-%, insbesondere bevorzugt 0,5 bis 3 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs
enthält.

20. Zubereitung nach Anspruch 16, **dadurch gekennzeichnet, dass** sie in Form einer Wasser-in-Öl-Emulsion vorliegt.

21. Zubereitung nach Anspruch 20, **dadurch gekennzeichnet, dass** sie als Emulsion vom Typ Wasser-in-Öl, vorzugsweise als kosmetische oder dermatologische Emulsion vom Typ Wasser-in-Öl, vorliegt und bezogen auf das Gesamtgewicht der Zubereitung
a) bis zu 95 Gew.-%, vorzugsweise 40 bis 95 Gew.-%, besonders bevorzugt 50 bis 90 Gew.-%, insbesondere bevorzugt 60 bis 85 Gew.-% einer Wasserphase,
b) bis zu bis zu 60 Gew.-%, vorzugsweise 2 bis 60 Gew.-%, besonders bevorzugt 5 bis 40 Gew.-%, insbesondere bevorzugt 10 bis 30 Gew.-% einer Ölphase,
c) bis zu 20 Gew.-%, vorzugsweise 0,5 bis 20 Gew.-%, besonders bevorzugt 1 bis 15 Gew.-%, insbesondere bevorzugt 4 bis 12 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs
enthält.

22. Zubereitung nach einem oder mehreren der Ansprüche 1 bis 21, **dadurch gekennzeichnet, dass** die Copolymerwachse in mikronisierter Form eingesetzt werden.

23. Zubereitung nach einem oder mehreren der Ansprüche 1 bis 22, **dadurch gekennzeichnet, dass** sie in Form einer Dispersion vorliegt und
a) ein Trägermaterial, vorzugsweise ein oder mehrere Ölkomponenten und/oder Lösungsmittel,
b) einen oder mehrere Emulgatoren und
c) neben den in Anspruch 1 genannten Copolymerwachsen gegebenenfalls ein oder mehrere weitere Wachse
enthält.

24. Zubereitung nach einem oder mehreren der Ansprüche 1 bis 23, **dadurch gekennzeichnet, dass** sie als dekoratives Mittel vorliegt.

25. Zubereitung nach einem oder mehreren der Ansprüche 1 bis 24, vorzugsweise in Form eines Puders, Preßlings, einer Paste, Creme oder eines Sticks, **dadurch gekennzeichnet, dass** sie ein oder mehrere Farbmittel, vorzugsweise ausgewählt aus Farblacken, Toner und Pigmenten, enthält.

26. Zubereitung nach einem oder mehreren der Ansprüche 1 bis 25, **dadurch gekennzeichnet, dass** sie einen oder mehrere UV-Lichtschutzfilter enthält, vorzugsweise ein Sonnenschutzmittel ist, und besonders bevorzugt in Form eines Sprays, Sticks, einer Paste, eines Gels oder einer Lotion vorliegt.

27. Zubereitung nach einem oder mehreren der Ansprüche 1 bis 26, **dadurch gekennzeichnet, dass** sie ein oder mehrere Antioxidantien enthält.

28. Zubereitung nach einem oder mehreren der Ansprüche 1 bis 23 und 25 bis 27, vorzugsweise in der Form eines Sprays, Sticks, einer Paste, eines Gels oder einer Lotion, **dadurch gekennzeichnet, dass** sie ein Deodorant oder Antiperspirant ist und ein oder mehrere Substanzen ausgewählt aus antimikrobiell wirkenden Substanzen, Adstringentien und deodorierenden Stoffen enthält.

29. Zubereitung nach Anspruch 28, **dadurch gekennzeichnet, dass** sie ein oder mehrere deodorierende Stoffe enthält und diese ausgewählt sind aus Allantoin und Bisabolol.

30. Zubereitung nach einem oder mehreren der Ansprüche 1 bis 23 und 25 bis 27, **dadurch gekennzeichnet, dass** es ein Peeling ist.
